# EUROPEAN PATENT SPECIFICATION

(11) **EP 3 943 614 B1**
(45) Date of publication and mention of the grant of the patent: **21.02.2024**
(21) Application number: 20795039.5
(22) Date of filing: 22.04.2020
(51) Int. Cl.: C12Q 1/6862, C12Q 1/6844, C12Q 1/6865

(54) **NOVEL PROBE SET FOR ISOTHERMAL ONE-POT REACTION, AND USES THEREOF**
NEUARTIGER SONDENSATZ ZUR ISOTHERMEN EINTOPFREAKTION UND VERWENDUNGEN DAVON
NOUVEAU JEU DE SONDES POUR UNE RÉACTION ISOTHERME MONOTOPE ET SES UTILISATIONS

(30) Priority: 22.04.2019 KR 20190046713
(43) Date of publication of application: 26.01.2022
(73) Proprietor: POSTECH Research and Business Development Foundation, Pohang-si, Gyeongsangbuk-do 37673 (KR)
(72) Inventor: LEE, Jeong Wook, Pohang-si Gyeongsangbuk-do 37673 (KR); JUNG, Gyoo Yeol, Pohang-si Gyeongsangbuk-do 37673 (KR); WOO, Chang Ha, Pohang-si Gyeongsangbuk-do 37655 (KR); JANG, Sungho, Yongin-si 17003 (KR); SHIN, Giyoung, Uiseong-gun Gyeongsangbuk-do 37362 (KR)
(74) Representative: Cabinet Becker et Associés
(86) International application number: PCT/KR2020/005331
(87) International publication number: WO 2020/218831

(56) References cited:
- KR-A- 20030 082 535
- KR-A- 20130 060 959
- KR-A- 20170 041 897
- KR-A- 20170 067 242
- KR-A- 20170 078 456
- RAMANI VIJAY ET AL: "High-throughput determination of RNA structure by proximity ligation", NATURE BIOTECHNOLOGY, NATURE PUBLISHING GROUP US, NEW YORK, vol. 33, no. 9, 1 September 2015 (2015-09-01), pages 980-984, XP037152140, ISSN: 1087-0156, DOI: 10.1038/NBT.3289 [retrieved on 2015-09-01]
- YONGXI ZHAO ET AL: "Isothermal Amplification of Nucleic Acids", CHEMICAL REVIEWS, vol. 115, no. 22, 9 November 2015 (2015-11-09), pages 12491-12545, XP055562529, US ISSN: 0009-2665, DOI: 10.1021/acs.chemrev.5b00428
- QIU LI-PING ET AL: "Highly Sensitive and Selective Bifunctional Oligonucleotide Probe for Homogeneous Parallel Fluorescence Detection of Protein and Nucleotide Sequence", ANALYTICAL CHEMISTRY, vol. 83, no. 8, 29 March 2011 (2011-03-29) , pages 3050-3057, XP55982181, US ISSN: 0003-2700, DOI: 10.1021/ac103274j
- GIBRIEL ABDULLAH A ET AL: "Advances in ligase chain reaction and ligation-based amplifications for genotyping assays: Detection and applications", MUTATION RESEARCH. REVIEWS IN MUTATION RESEARCH, ELSEVIER, AMSTERDAM, NL, vol. 773, 2 May 2017 (2017-05-02), pages 66-90, XP085174532, ISSN: 1383-5742, DOI: 10.1016/J.MRREV.2017.05.001

## Description

### [Technical Field]

The present invention relates to a novel probe set for isothermal one-pot reaction and uses thereof. Specifically, a method is provided for easily, accurately, and rapidly molecular diagnosis in the field, having a form applicable in the field based on a nucleic acid sequence using a single reaction composition under isothermal conditions.

### [Background Art]

Methods for detecting specific nucleic acids (DNA or RNA) or proteins are fundamentally critical techniques in the field of scientific research. Specific nucleic acids or proteins have been detected and identified to allow researchers to determine which genetic and biological markers are indicative of human health.

Such a method for detecting a nucleic acid or protein is used to detect a target gene, such as a pathogen gene and its variant or the expression of a specific gene, present in a sample.

Meanwhile, as income levels increase in modern times, interest in health and hygiene increases, but it is not easy to directly detect microorganisms invisible and quantitatively evaluate them.

Various microorganisms live in everyday objects such as hair combs, cell phones, desks, clothes, etc., used by humans, spaces such as toilets and bedrooms and even the general air. These microorganisms may contain opportunistic and pathogenic bacteria.

As a traditional method for measuring the number of microorganisms, the standard plate method is used in which the sample collected from the environment is serially diluted and spread on a medium capable of culturing microorganisms, and the number of colonies generated from the medium is calculated after 2-3 days to estimate the amount thereof. These traditional methods require specialized experimental tools and skilled experimenters as well as take a very long time, making it difficult for the general public to quickly measure viable microorganisms.

Recently, several methods have been developed in order to solve the problems of the traditional method. It is to indirectly measure the number of microorganisms by measuring the number of various constituents that are always present in the cell.

A representative method includes a method of measuring based on the amount of ATP. ATP is used as a major source of bioenergy in cells and is a useful substance for measuring the number of cells in a sample as a component common to all living things. Due to these advantages, the ATP measurement method is currently widely used for the quantitative measurement of microorganisms. In order to measure ATP, a luminescent reaction is generally induced using a luciferase enzyme and quantified through light intensity. In this case, luciferin is used as a substrate.

However, the method has disadvantages in that ATP in the reaction sample is rapidly depleted, so that the signal does not last for a long time, and the production cost of the added enzyme and substrate is high.

Further, the component for measurement is an enzyme so that it has a limitation in storage ability. There is another problem in the method for quantifying microbial cells through ATP measurement. Even when cells die, the ATP function is maintained, so it is highly likely that the killed or very low-viability microorganisms will also be measured.

Another common method for diagnosing pathogenic microorganisms includes antibody-based techniques such as enzyme-linked immunosorbent assay (ELISA) or immunoradiometric assay (IRMA). However, these methods are also not suitable as a method for diagnosing infectious diseases because it takes 2-3 months to generate an antibody that responds to a specific antigen so that variants thereof are frequent and spread in a short time.

Therefore, instead of antibody-based diagnostic technology, a molecular level diagnostic technology capable of determining by examining nucleic acids such as DNA and RNA containing trace amounts of information on infectious substances is suitable for diagnosing infectious diseases.

Among these molecular diagnostic techniques, there is a method using a ligase. When a specific DNA fragment is put into the sample, the ligase present in the cell recognizes it and connects the two fragments through a polymerization reaction. It is known that the number of microorganisms can be evaluated when real-time PCR analysis is performed using specific primers on the fragments connected in this manner.

However, since RT-PCR, PCR, and gel electrophoresis are required to identify a new nucleic acid molecule created through the reaction of ligase with a DNA strand, expensive equipment and skilled experimenters are required, and the analysis time is very long. Further, ATP molecules are present in dead cells, so it is not possible to measure only living microorganisms like the luciferase assay.

In other words, molecular diagnostic technology using these nucleic acids has superior sensitivity compared to conventional diagnostic methods, so it is possible to detect a specific gene to prevent diseases or to perform screening tests, early identification, and rapid response to prevent infectious diseases. However, most molecular diagnostic technology using nucleic acid has the disadvantage that it requires temperature-circulation nucleic acid amplifier (thermocycler) equipment and a professional capable of handling the machine.

Accordingly, there is a need to develop methods capable of reducing the time consumption required to generate an antibody by binding to a specific antigen, which is a problem with the conventional antibody-based diagnostic technology described above, and very effectively and accurately detecting and/or diagnosing the target DNA/RNA sequence within a short time without securing expensive equipment and skilled manpower.

### [Disclosure]

### [Technical Problem]

Under these circumstances, the present inventors have made intensive research efforts to develop a technology capable of detecting single or multiple target nucleic acid sequences more simply and without false positive and negative results while addressing the issues of the prior art. As a result, the present inventors have constructed specific promoter sequences; and a first DNA probe that is a promoter probe (PP) consisting of a sequence that hybridizes with a target gene, a sequence that hybridizes with the target gene; and a second DNA probe, which is a reporter probe (RP) consisting of a sequence encoding a fluorescent-label RNA aptamer. In addition, The three steps of the ligation reaction of the DNA probes using the target gene as a splint, the transcription process by RNA polymerase from the ligated single-stranded DNA, and the fluorescence reaction in which the aptamer structure in the transcription product generated in the transcription process binds to specific chemical molecules are unified under isothermal conditions so that multiple steps are performed in a one-pot reaction, particularly, without an amplification step. Thus, the present invention was completed by identifying that it may be a platform for not only easily and conveniently detecting a target gene, but also actually detecting highly pathogenic microorganisms singly or plurally to diagnose highly pathogenic diseases requiring rapid response.

Accordingly, one object of the present invention is to provide a probe set of an isothermal one-pot reaction for detecting a target nucleic acid sequence.

Another object of the present invention is to provide a composition for detecting a target nucleic acid sequence including the probe set.

Still another object of the present invention is to provide a kit for detecting a target nucleic acid sequence.

Yet another object of the present invention is to provide a method for detecting a target nucleic acid sequence under isothermal one-pot reaction conditions without a separate amplification reaction.

Further another object of the present invention is to provide a molecular diagnostic method for testing at on-site under isothermal one-pot reaction conditions without a separate amplification reaction.

Other objects and advantages of the present invention will become more apparent from the following appended claims and drawings.

### [Technical Solution]

The terms used in the present specification are used for the purpose of description only and should not be construed as limiting. The singular expression includes the plural expression unless the context clearly dictates otherwise. It should be understood that terms such as "comprise" or "have" used in the present specification are intended to designate that a feature, number, step, operation, component, part, or a combination thereof described in the specification exists, but does not preclude the existence or addition of at least one feature, number, step, operation, component, part, or a combination thereof

Unless defined otherwise, all terms used herein, including technical or scientific terms, have the same meaning as commonly understood by one of ordinary skill in the art to which the embodiment belongs. Terms such as those defined in a commonly used dictionary should be interpreted as having a meaning consistent with the meaning in the context of the related art and should not be interpreted in an ideal or excessively formal meaning unless explicitly defined in the present application.

Hereinafter, the present invention is described in detail.

According to an aspect of the present invention, the present invention provides a probe set of an isothermal one-pot reaction for detecting a target nucleic acid sequence, the probe set comprising a first probe and a second probe,
wherein the first probe is a promoter probe (PP) having a structure represented by the following general formula I;

   3'-X-Y-5' (I)
wherein, X represents a stem-loop structure portion having a promoter sequence that may be recognized by RNA polymerase; Y represents an upstream hybridization sequence (UHS) portion having a hybridization sequence complementary to the target nucleic acid sequence; the target nucleic acid sequence is DNA or RNA; and X and Y are deoxyribonucleotides;
wherein the second probe is a reporter probe (RP) having a structure of represented by the following general formula II;

   3'-Y'-Z-5' (II)
wherein Y' represents a downstream hybridization sequence (DHS) portion having a hybridization sequence complementary to the target nucleic acid sequence; Z represents an aptamer sequence portion having a label or an interactive label system containing a plurality of labels to generate a detectable signal; the target nucleic acid sequence is DNA or RNA; and Y' and Z are deoxyribonucleotides; and
wherein the first probe and the second probe are hybridized with the target nucleic acid sequence to allow ligation of the first probe and the second probe; and transcription of the ligation product is initiated by RNA polymerase to generate a signal.

The technology of the present invention using the probe set of the present invention is called "isothermal one-pot reaction platform" or "molecular diagnosis platform using ligation reaction."

Each of the first probes of the probe set of the present invention has a structure including two different intrinsic portions in one oligonucleotide molecule:
X, which is a portion of a stem-loop structure having a promoter sequence that can be recognized by RNA polymerase; and Y, which is a portion of an upstream hybridization sequence (UHS) having a hybridization sequence complementary to the target nucleic acid sequence.

Each of the second probes of the probe set of the present invention has a structure including two different intrinsic portions in one oligonucleotide molecule:
Y', which is a portion of a downstream hybridization sequence (DHS) having a hybridization sequence complementary to the target nucleic acid sequence; and Z, which is a portion of an aptamer sequence having a label or an interactive label system containing a plurality of labels to generate a detectable signal.

This structure enables the probe set of the present invention to become a probe that exhibits a high detection effect in a short time under isothermal one-pot reaction conditions as a unified step.

More specifically, the probe design of the present invention allows two single-stranded DNA probes to expose the target recognition sequence, causing hybridization of the target RNA/DNA and the probe set at isothermal (e.g., a temperature at which the enzyme can act). Hybridization sequences are designed to maximize hybridization to the target RNA/DNA while minimizing the formation of any other structures. An efficient hybridization process between the probe and target RNA/DNA allows for high sensitivity during isothermal reactions.

The first probe, the promoter probe, is designed to form a stem-loop structure, and the stem portion forms a double-stranded RNA polymerase promoter sequence to initiate a transcription process using RNA polymerase. Since the double-stranded RNA polymerase promoter portion is physically linked by the loop sequence, the probability that the double-stranded promoter is formed into a functional form is higher than when the double-stranded promoter is not linked by the loop sequence. Therefore, the self-assembled promoter sequence in which the hairpin structure is formed in the promoter probe may effectively promote the hybridization process and the subsequent transcription process.

The second probe, the reporter probe, is designed to include an aptamer sequence as a reporter, and the final product may be identified by the aptamer that generates a signal in response to a specific substance.

In the present invention, "aptamer" used as a reporter is a single-stranded nucleic acid (DNA, RNA or modified nucleic acids) that has a stable tertiary structure and can bind to a target molecule with high affinity and specificity.

After the aptamer discovery technology called systematic evolution of ligands by exponential enrichment (SELEX) was developed, aptamers capable of binding to various target molecules, such as small molecule organic matter, peptides, and membrane proteins, have been continuously discovered. Aptamers are often compared to single antibodies because of their inherent high affinity (usually at pM level) and specificity to bind to a target molecule and have high potential as an alternative antibody, especially as a "chemical antibody." Further, the absorption wavelength band and the emission wavelength band are different depending on the binding material so that the binding of an aptamer and a specific chemical molecule may be confirmed by a method such as expressing fluorescence.

As long as the aptamer of the present invention and the reactant interacting with the aptamer generate a detectable signal as a target effect, any kind of aptamer and reactant may be used.

After hybridization, the first probe and the second probe hybridized to the target nucleic acid sequence are ligated.

That is, after the first probe and the second probe of the present invention are hybridized with the target nucleic acid sequence, a nick formed between the two probes is ligated using a ligation agent.

According to a preferred embodiment of the present invention, the first probe and the second probe are positioned at immediately adjacent locations to each other when hybridized with the target nucleic acid sequence.

The adjacent positioning is necessary for ligation reactions between the two probes. The term used herein "adjacent" in conjunction with hybridization positions of the first probe and the second probe means that the 3'-end of one probe and the 5'-end of the other probe are sufficiently near each other to allow connection of the ends of both probes to one another.

Since enzymatic ligation is the preferred method of covalently attaching the first probe and the second probe, the term "ligation" will be used throughout the application.

However, the term "ligation" is a general term and is to be understood to include any method of covalently attaching both probes.

The ligation reaction of the present invention may be performed using a wide variety of ligation agents, including enzymatic ligation agents and non-enzymatic ligation agents (e.g., chemical agents and photoligation agents).

Chemical ligation agents include, without limitation, activating, condensing, and reducing agents, such as carbodiimide, cyanogen bromide (BrCN), N-cyanoimidazole, imidazole, 1-methylimidazole/carbodiimide/cystamine, dithiothreitol (DTT) and ultraviolet light.

Autoligation, i.e., spontaneous ligation in the absence of a ligating agent, is also within the scope of the teachings herein. Detailed protocols for chemical ligation methods and descriptions of appropriate reactive groups can be found in Xu et al., Nucl. Acids Res., 27:875-81(1999); Gryaznov and Letsinger, Nucl. Acids Res. 21:1403-08(1993); Gryaznov et al., Nucleic Acid Res. 22:2366-69(1994); Kanaya and Yanagawa, B 5 iochemistry 25:7423-30(1986); Luebke and Dervan, Nucl. Acids Res. 20:3005-09(1992); Sievers and von Kiedrowski, Nature 369:221-24(1994); Liu and Taylor, Nucl. Acids Res. 26:3300-04(1999); Wang and Kool, Nucl. Acids Res. 22:2326-33(1994)).

Photoligation using light of an appropriate wavelength as a ligation agent is also within the scope of the teachings. In certain embodiments, photoligation comprises probes comprising nucleotide analogs, including but not limited to, 4-thiothymidine (s4T), 5-vinyluracil and its derivatives, or combinations thereof.

According to a preferred embodiment of the present invention, the ligation reaction is occurred by an enzymatic ligation agent. The ligation agent includes one selected from the group consisting of SplintR ligase, bacteriophage T4 ligase, *E. coli* ligase, Afu ligase, Taq ligase, Tfl ligase, Mth ligase, Tth ligase, Tth HB8 ligase, Thermus species AK16D ligase, Ape ligase, LigTk ligase, Aae ligase, Rm ligase, Pfu ligase, ribozyme and variants thereof.

The internucleotide linkage generated by the ligation includes phosphodiester bond and other linkages. For instance, the ligation using ligases generally produces phosphodiester bonds.

Non-enzymatic methods for ligation may form other internucleotide linkages. Other internucleotide linkages include, without limitation, covalent bond f 5 ormation between appropriate reactive groups such as between an α-haloacyl group and a phosphothioate group to form a thiophosphorylacetylamino group, a phosphorothioate and tosylate or iodide group to form a 5'-phosphorothioester, and pyrophosphate linkages.

After the ligation reaction, the resulting ligation product includes an aptamer sequence and becomes single-stranded DNA as a template for amplifying the target RNA/DNA.

Subsequently, when the transcriptional process is initiated by RNA polymerase, the target RNA/DNA is elongated from the single-stranded DNA, which is a template for amplifying the target RNA/DNA containing the aptamer sequence such that signals are generated quickly and simply by the aptamer which binds to specific chemical molecules to fluoresce.

Further, when the RNA aptamer is used as a reporter, the time it takes to observe the generated signal is shortened, compared to a conventional fluorescent signal using a fluorescent protein.

If the first probe and the second probe are not performed as described above, a signal emitted from the label on the transcriptional product of the first probe and the second probe is not finally generated, and thus the target nucleic acid sequence is not detected.

The RNA polymerase of the present invention may include any kind of RNA polymerase as long as it can recognize the promoter portion so as to initiate transcription as the desired effect.

Preferably, the RNA polymerase may be selected from the group consisting of bacteriophage T7 RNA polymerase, bacteriophage T3 polymerase, bacteriophage RNA polymerase, bacteriophage ΦII polymerase, *Salmonella* bacteriophage SP6 polymerase, *Pseudomonas* bacteriophage gh-1 polymerase, *E. coli* RNA polymerase holoenzyme, *E. coli* RNA polymerase core enzyme, human RNA polymerase I, human RNA polymerase II, human RNA polymerase III, human mitochondrial RNA polymerase and variants thereof, but is not limited thereto.

The bacteriophage T7 RNA polymerase is used in one embodiment of the present invention.

Likewise, as long as transcription may be initiated by RNA polymerase, any promoter sequence known in the art may be used for the promoter region in the first probe of the present invention recognized by RNA polymerase.

The ligation agent and the polymerase may be appropriately adjusted to optimize the isothermal one-pot reaction of the present invention. They are included preferably 1 : 1 to 1 : 5 units, more preferably 1 : 4 units, most preferably 1 : 1 units in a one-pot reaction buffer.

According to a preferred embodiment of the present invention, the label may be selected from the group consisting of a chemical label, an enzymatic label, a radioactive label, a fluorescent label, a luminescent label, a chemiluminescent label, and a metal label.

The isothermal one-pot reaction of the present invention, without a separate amplification reaction, is simultaneously performed, which is unified at any one of the designated temperatures in the range of 15°C to 50°C.

The temperature in the range of 15°C to 50°C is a temperature known in the art for enzymes to act, and is not limited thereto, as long as the desired effect of the present invention may be obtained.

In an embodiment of the present invention, preferably, the specified temperature is 37°C.

Further, the isothermal one-pot reaction is performed simultaneously with a single reaction buffer containing Tris-HCl, MgCl₂, NTPs, NaCl and ET-SSB (extreme thermostable single-stranded DNA binding protein).

In the present invention, the single reaction buffer preferably includes 1 to 100 mM Tris-HCl; 1 to 50 mM MgCh; 0.1 to 10 mM NTPs; 1 to 50 mM NaCl; and 1 to 800 ng ET-SSB, more preferably 10 to 60 mM Tris-HCl; 1 to 30 mM MgCh; 0.5 to 5 mM NTPs; 1 to 30 mM NaCl; and 100 to 600 ng ET-SSB, most preferably 50 mM Tris-HCl; 10 mM MgCh; 1 mM NTPs; 10.5 mM NaCl; and 400 ng ET-SSB,

According to another aspect of the present invention, the present invention provides a method for detecting a target nucleic acid sequence under isothermal one-pot reaction conditions without a separate amplification reaction, the method including the following steps of:
(a) treating a sample with the probe set of an isothermal one-pot reaction for detecting a target nucleic acid sequence including the first probe and the second probe described above to hybridize with the target nucleic acid sequence;
(b) treating the hybridization product of step (a) with a ligation agent to ligate the first probe and the second probe of the probe set, and treating the ligation product with a polymerase to initiate transcription; and
(c) treating the transcription product of step (b) with an aptamer-reactive substance to detect aptamer signal generation in the transcription product, in which the signal generation indicates the presence of a target nucleic acid sequence in the sample.

The isothermal one-pot reaction of the present invention, without a separate amplification reaction, is performed simultaneously in which the temperature is unified at any one of the designated temperatures in the range of 15°C to 50°C, and preferably, the specified temperature is 37°C.

The isothermal one-pot reaction is performed simultaneously with a single reaction buffer containing Tris-HCl, MgCl₂, NTPs, NaCl and ET-SSB (extreme thermostable single-stranded DNA binding protein).

In addition, the present invention is characterized in that an additional separate amplification process (e.g., PCR) is not required, and the amplification process is performed automatically during an isothermal one-pot reaction.

The reason why an isothermal one-pot reaction including such an amplification process is possible is because of (i) the design of the second probe in which an aptamer is formed downstream so that a ligation reaction occurs with the first probe including the double-stranded promoter sequence having a hairpin structure and (ii) the amplification process that occurs naturally in the reaction without a separate amplification process by using what may be used as a target sequence (i.e., splint) when the ligated product is transcribed by transcription initiation.

That is, when the transcription initiation occurs from the RNA polymerase promoter sequence of the first probe in which the ligation product of the ligation reaction between the first probe and the second probe forms a hairpin structure to form a transcript, the transcripts contain the same sequence as the target RNA sequence so that it may be used as the target RNA. In addition, since the transcribed ligation product includes the same sequence as the target nucleic acid sequence, it may act as a target RNA among target nucleic acids.

Therefore, the platform using the probe set of the present invention is designed so that ligation, transcription reaction, and fluorescence reaction occur simultaneously. Further, the transcription product in which the transcription reaction of the ligated product has occurred is used as a splint RNA, so that a single isothermal reaction containing amplification process is included.

Accordingly, steps (a) to (c) of the present invention may be performed simultaneously in one vessel, such as a tube.

In the present invention, the sequence of the DNA probe is not limited to the sequence described in the Examples of the present invention as long as the target effect is achieved. Further, it is applicable to all target gene sequences. In addition, the aptamer used for final signal confirmation is not limited to the malachite green aptamer, and any type of RNA-based fluorescent aptamer may be used.

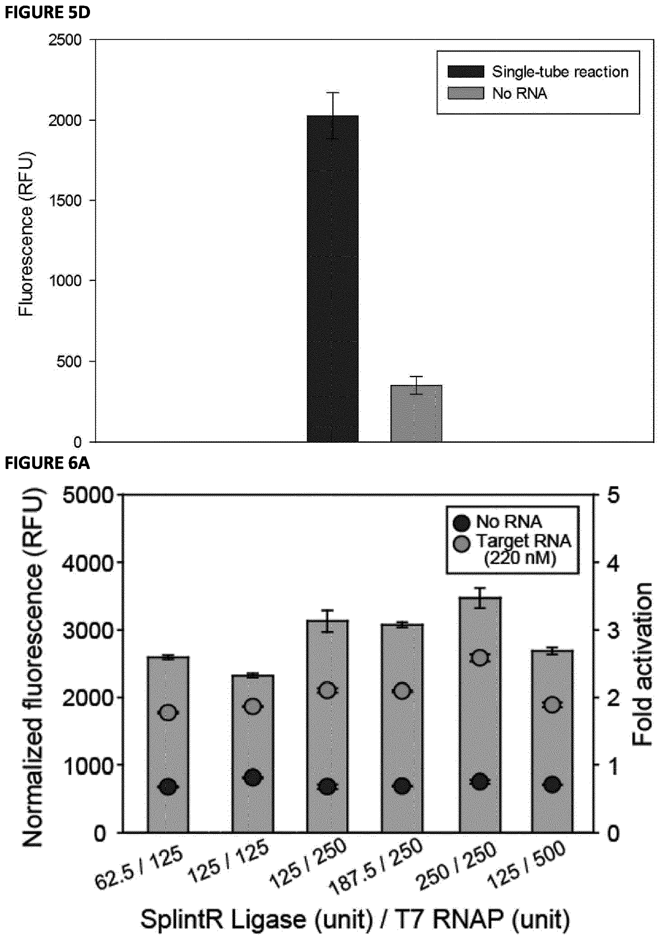

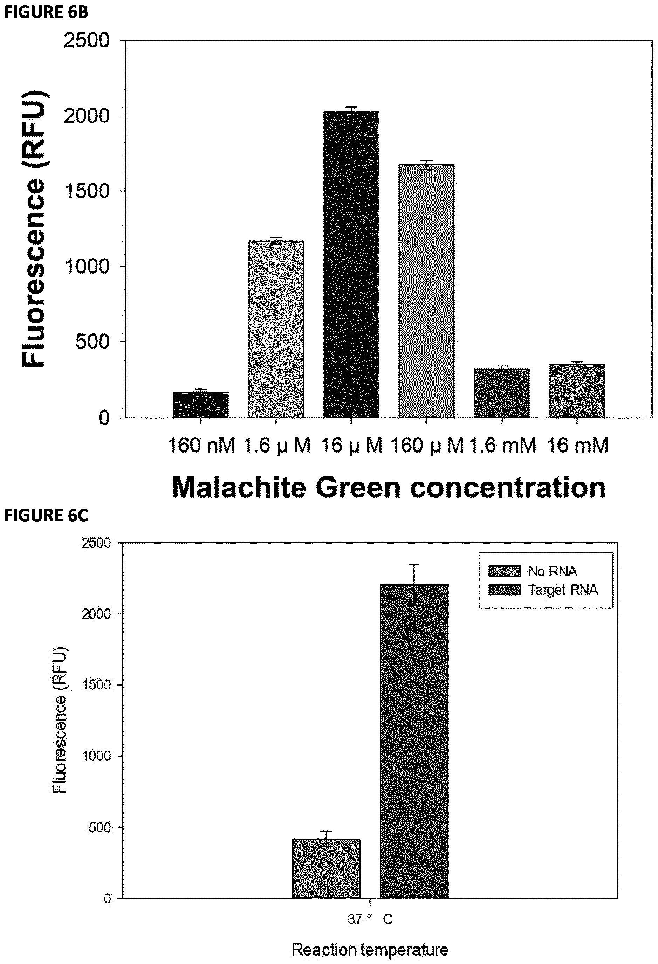

According to an embodiment of the present invention, provided is a molecular diagnostic method capable of determining whether or not a specific disease is infected, the method including steps of 1) ligating using the prepared two DNA probes, target RNA, and SplintR ligase, 2) allowing the transcription of the ligated probe assembly through T7 RNA polymerase to prepare transcription product and 3) confirming whether an aptamer present downstream of the generated transcription product binds to a specific chemical molecule and it is detected with the intensity of fluorescence. A schematic for this is shown in FIG. 6.

According to another aspect of the present invention, the present invention provides a composition for detecting a target nucleic acid sequence including an isothermal one-pot reaction probe set for detecting a target nucleic acid sequence, the probe set including the first probe and the second probe described above; a ligation agent; a polymerase; and an isothermal one-pot reaction buffer.

According to a preferred embodiment of the present invention, the composition may include two or more isothermal one-pot reaction probe sets for detecting target nucleic acid sequences.

Each of the two or more isothermal one-pot reaction probe sets for detecting the target nucleic acid sequences includes different interactive labeling systems; each of the two or more probe sets binds to different target nucleic acid sequences; and due to this, multiple detections of different target nucleic acid sequences are possible.

That is, the two or more types of targets may be interactively different molecular diagnostic objects, for example, pathogenic microorganisms. In this case, it is possible to simultaneously detect and diagnose different pathogens.

Further, the two or more types of targets may be different target sites present in a single pathogen. Thus, in this case, different sites of the target are effectively detected to enable a more accurate and precise diagnosis.

The composition of the present invention is prepared to perform the detection of a target nucleic acid sequence by the probe set of the present invention as described above. Thus, the overlapping contents are excluded in order to avoid the complexity of the present specification.

According to another aspect of the present invention, the present invention provides a kit for detecting a target nucleic acid sequence, the kit including the composition for detecting a target nucleic acid sequence described above.

The kit of the present invention as described above may additionally include various polynucleotide molecules, enzymes, buffers and reagents. In addition, the kit of the present invention may include reagents necessary for carrying out the positive control and negative control reactions. The optimal amount of reagent to be used in any one particular reaction may be readily determined by one of ordinary skill in the art having the teachings in the present specification.

Typically, kits of the present invention are prepared as separate packages or compartments including the aforementioned components.

The kit of the present invention is manufactured to perform the detection of a target nucleic acid sequence by the probe set of the present invention as described above. Thus, overlapping contents are excluded in order to avoid the complexity of the present specification.

According to another aspect of the present invention, the present invention provides a molecular diagnostic method for testing at on-site under isothermal one-pot reaction conditions, without a separate amplification reaction, the method including the following steps of:
(a) treating a sample with the probe set of an isothermal one-pot reaction for detecting a target nucleic acid sequence including the first probe and the second probe described above to hybridize with the target nucleic acid sequence;
(b) treating the hybridization product of step (a) with a ligation agent to ligate the first probe and the second probe of the probe set, and treating the ligation product with a polymerase to initiate transcription; and
(c) treating the transcription product of step (b) with an aptamer-reactive substance to detect aptamer signal generation in the transcription product, in which the signal generation indicates the presence of a target nucleic acid sequence in the sample.

The kind of target is not limited as long as it may be diagnosed by the method of the present invention.

In the present invention, the target is preferably a pathogenic microorganism.

The pathogenic microorganism may include at least one selected from the group consisting of *Staphylococcus Aureus, Vibrio vulnificus, E. coli,* middle east respiratory syndrome coronavirus, influenza A virus, severe acute respiratory syndrome coronavirus, respiratory syncytial virus (RSV), human immunodeficiency virus (HIV), herpes simplex virus (HSV), human papillomavirus (HPV), human parasite influenza virus (HPIV), dengue virus, hepatitis B virus (HBV), yellow fever virus, rabies virus, Plasmodium, cytomegalovirus (CMV), *Mycobacterium tuberculosis, Chlamydia trachomatis,* Rota virus, human metapneumovirus (hMPV), Crimean-Congo hemorrhagic fever virus, Ebola virus, Zika virus, Henipavirus, Norovirus, Lassavirus, Rhinovirus, Flavivirus, Rift valley fever virus, hand-foot-and-mouth disease virus, *Salmonella* sp., *Shigella* sp., *Enterobacteriaceae* sp., *Pseudomonas* sp., *Moraxella* sp., *Helicobacter* sp. and *Stenotrophomonas* sp.

In the method of the present invention, an isothermal one-pot reaction probe set for detecting two or more types of target nucleic acid sequences may be designed to quickly and accurately detect two or more types of target nucleic acid sequences in the field using the design.

In one embodiment of the present invention, the present inventors have used and applied only a minimal design based on a highly modular probe structure to 6 pathogens, thereby successfully multi-detecting and diagnosing these pathogens.

Accordingly, the probe set of the present invention may be designed from any RNA/DNA as long as the length of the target nucleic acid sequence is appropriate. Thus, this characteristic allows the set to respond quickly to the outbreak of infectious disease and provides a significant advantage over antibody-based diagnosis.

In other words, the target sequence detection platform using the probe set of the present invention serves as a powerful diagnostic platform for RNA/DNA detection of a target to provide a short diagnosis time, high sensitivity and specificity and a simple analysis procedure. Further, it may be a suitable diagnostic method for new infectious diseases that require rapid response without expensive equipment and diagnostic experts.

Further, the probe set of the first probe and the second probe of the present invention having such a highly modular structure is completely free from false-positive and false-negative results in multi-target detection.

Since the method of the present invention includes the probe set of the present invention described above, redundant descriptions are excluded to avoid the excessive complexity of the present specification.

### [Advantageous Effects]

The molecular diagnostic platform according to the present invention is used to quickly and accurately detect pathogens even when the target gene is present at a very low concentration. In addition, the elements (reaction buffer, enzyme) required in the diagnostic process are much simpler and uncomplicated than the conventional antibody-based diagnostics, thus anyone can proceed with the process. All reactions proceed simultaneously at isothermal temperature without the expensive equipment used in general nucleic acid-based molecular diagnostic technology, thereby increasing the sensitivity and speed of diagnosis.

### [Description of Drawings]

FIG. 1A shows the structures of two DNA probes of the present invention, FIG. 1B shows a ligation reaction using SplintR ligase, and FIG. 1C shows three key reactions of the ligation-based molecular diagnostic method of the present invention, and FIG. 1D shows the overall schematic of the method for detecting nucleic acids using the ligation reaction of the present invention.
FIG. 2 shows the result of confirming the ligation reaction between the two probes by capillary electrophoresis in the presence of the target RNA.
FIG. 3 shows the result of agarose gel electrophoresis to confirm the transcription product produced by the ligation reaction product of the present invention has been transcribed through the transcription process.
FIG. 4 shows the result of measuring the fluorescence value by adding the target material, malachite green, in order to confirm whether the transcription product correctly forms the malachite green aptamer structure.
FIG. 5A shows a schematic diagram of a nucleic acid detection platform consisting of an isothermal one-pot reaction of the present invention.
FIG. 5B shows the results of measuring fluorescence values in order to find a suitable reaction buffer for a one-pot reaction among 20 single reaction buffer candidate groups.
FIG. 5C shows the difference in fluorescence values shown by further adding protein (ET-SSB) to the selected reaction buffer (reaction buffer No. 1) in order to increase the efficiency of ligation.
FIG. 5D shows that the single reaction buffer of the present invention is practically suitable for carrying out a series of reactions (ligation, transcription reaction, and fluorescence reaction) in one tube.
FIG. 6A shows the results of finding the optimal amount of enzymes by controlling the amounts of enzymes (SplintR ligase and T7 RNA polymerase) required for the optimization of a one-pot reaction.
FIG. 6B shows the result of optimizing the amount of malachite green mediating the fluorescence reaction in a one-pot reaction.
FIG. 6C shows the result of confirming whether the target RNA can be detected at 37°C for the isothermal one-pot reaction.
FIG. 7A shows the detection result (gel image) by the probe set of the present invention when DNA is used as a splint.
FIG. 7B shows the detection result (electropherogram) by the probe set of the present invention when DNA is used as a splint
FIG. 7C shows the result of displaying fluorescence signals of transcription products binding to malachite green by the probe set of the present invention when DNA is used as a splint.
FIG. 7D shows the results of the probe set of the present invention in the case of using DNA as a splint in one vessel as a single reaction buffer under an isothermal temperature of 37°C.
FIG. 8A shows the results of the diagnosis rapidity of how long it takes for a target RNA to be detected through a reaction proceeding as an isothermal one-pot reaction.
FIG. 8B shows the results showing the detection limit through the change in the fluorescence value according to the target RNA concentration through a reaction proceeding as an isothermal one-pot reaction.
FIG. 9 (FIGS. 9A to 9F) shows the results showing the intensity of fluorescence values according to the target RNA concentration of various pathogens through a reaction proceeding as an isothermal one-pot reaction.
FIG. 10 (FIGS. 10A to 10D) shows the results of whether the cells of the actual pathogen are directly detected through the reaction proceeding as an isothermal one-pot reaction.
FIG. 11 (FIGS. 11A to 11C) shows the results of whether two different pathogens can be independently detected with different fluorescence signals and intensities through a reaction proceeding as an isothermal one-pot reaction.
FIG. 12 (FIGS. 12A to 12C) shows the results of whether different target sites of a specific pathogen can be detected with different fluorescence signals and intensities through a reaction proceeding as an isothermal one-pot reaction.

### [Modes of the Invention]

Hereinafter, the present invention is described in more detail through Examples. These Examples are only for illustrating the present invention. It will be apparent to those of ordinary skill in the art that the scope of the present invention is not to be construed as being limited by these Examples.

### Example 1. Validation of molecular diagnostic platform using ligation reaction

### 1-1. Ligation reaction using ligase

The present inventors have developed a molecular diagnostic platform capable of detecting a trace amount of target RNA using a ligation reaction in order to quickly, inexpensively, and accurately detect a target (e.g., a specific disease).

Therefore, in this Example, a ligation reaction by ligase was used based on a specially prepared DNA probe structure and sequence. In the present invention, the special DNA probe was designed to enable all three reactions of ligation, subsequent transcription, and aptamer structure formation and fluorescence expression.

For this, the following three conditions shall be satisfied.

First, since all reactions must be carried out at an isothermal temperature (e.g., 37°C), the polymerization reaction between the probe and the target RNA sequence must be sufficiently occur at this temperature, and undesired structures must not be formed during the process.

Second, the region on the DNA probe that polymerizes with the target RNA must not form an unnecessary structure and must exist as a single strand. Further, as transcription is initiated by RNA polymerase after the ligation reaction, a double-stranded promoter must be present on the DNA probe.

Third, the aptamer positioned at downstream must form an independent structure without interacting with upstream hybridization sequences.

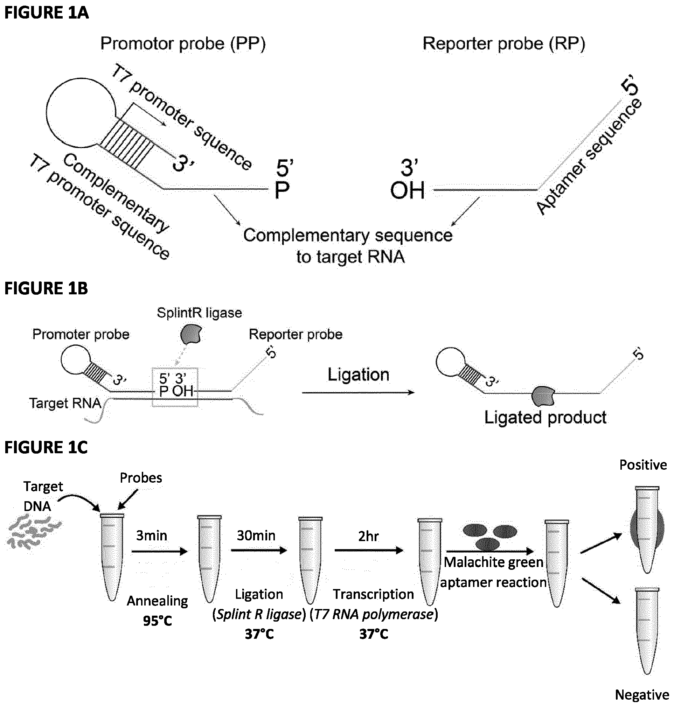

The structure and reaction of the DNA probe set satisfying the three conditions described above are shown in FIGS. 1A and 1B.

The above-described DNA probe set consists of two probes, and the platform consists of three core components as follows (FIG. 1C):
(1) Ligation reaction between probes using ligase
   - When DNA probes and target RNA are present, the target RNA is used as a splint to ligate the probe set consisting of two types of DNA probes. In other words, the two types of DNA probes complementary to the target RNA sequence are prepared, and they are ligated each other by the ligation using ligase (SplintR ligase).
*(2) In vitro* transcription reaction of ligated product
   - T7 RNA polymerase is attached to the T7 promoter sequence located at the 3' portion of the ligated product to proceed transcription process, thereby producing RNA as a transcript from the ligated product.
(3) Fluorescence expression using RNA aptamer
   - An aptamer located at the 3' end of the RNA transcript binds to a specific chemical, resulting in generation of a fluorescence signal such that the fluorescence signal is indicative of the presence of the target RNA.

In the present Examples, it is intended to diagnose MRSA infection through the core components of (1) - (3) as an embodiment.

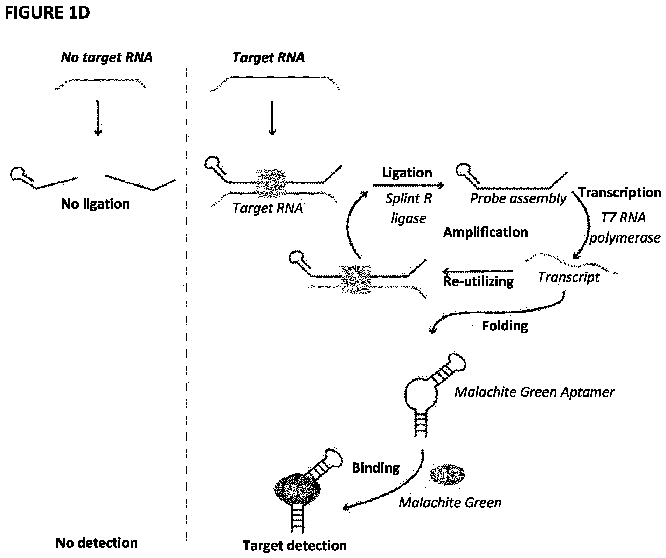

Since these processes are completed within 2 hours, the results may be quickly derived. After reaction (1) occurs, subsequent processes may be performed one after the other. If all the reactions (1) to (3) are completed, the desired result may be obtained. Thus, the accuracy of the diagnosis may be trusted (FIG. 1D).

The DNA probe set consists of the following two types of probes:
a first DNA probe (referred to as a promoter probe (PP)), in which the PP consists of two portions: a promoter sequence and an upstream hybridization sequence (UHS) that hybridizes with a target gene;
a second DNA probe (referred to as reporter probe (RP)), in which the RP consists of two portions: the downstream hybridization sequence (DHS) that hybridizes with the target gene and a sequence encoding fluorescent-reactive RNA aptamer as a reporter that can identify the final product.

In Example 1, *mecA,* a target gene of methicillin resistant *Staphylococcus aureus* (MRSA) was used as the target RNA (splint RNA); T7 promoter sequence was used as a promoter sequence; and a malachite green RNA aptamer was used as a fluorescent-reactive RNA aptamer.

Detailed sequences of the two types of DNA probes used in Example 1 are shown in Table 1 below.

**[Table 1]**

| | |
|---|---|
| PP (UHS + T7 promoter complementary + loop + T7 promoter) | 5'- TTCTCCTTGTTTCATTTTGAGTTCTGCAG ccctatagtgagtcgtattaggatccacaacaggatcctaatacga ctcactatagggg-3' (SEQ ID NO.: 1) |
| RP (Malachite Green Aptamer + DHS) | 5'- ggatccattcgttacctggctctcgccagtcgggatccaccAC CCAATTTGTCTGCCAGT-3' (SEQ ID NO.: 2) |

| | |
|---|---|
| (Nucleotides indicated in capital letters refer to hybridization sequences complementary to the target nucleic acid sequence. The nucleotides indicated in lower case letters refer to the T7 promoter complementary sequence + the loop sequence + the T7 promoter sequence, constituting the stem-loop structure. The underlined nucleotides refer to nucleotides constituting the stem in the stem-loop structure. Nucleotides indicated in lower case italics refer to aptamer sequences.) | |

Briefly, the previously prepared two DNA probes and target RNA were denatured with heat at 95°C for 3 minutes in a 10x annealing reaction buffer [100 mM Tris-HCl (pH 7.4), 500 mM KCl], and then slowly cooled at room temperature. The target RNA was cloned by inserting the T7 promoter sequence into the upstream of the sequence of the *mecA* DNA, which is the target gene of methicillin resistant *Staphylococcus aureus* (MRSA). The T7 promoter sequence was inserted upstream of the DNA sequence, and then were transcribed using T7 RNA polymerase (New England Biolabs., Ipswich, USA). RNA obtained from the transcription reaction was treated with DNaseI (Takara Bio Inc., Nojihigashi, Japan) and then purified using RiboClear (GeneAll Biotechnology, Seoul, Korea).

As a conventionally known method, the annealed oligonucleotides were added to 10x SplintR ligase reaction buffer [50 mM Tris-HCl (pH7.5), 10 mM MgCl₂, 1 mM ATP, 10 mM DTT, 15 mM NaCl] with SplintR ligase (New England Biolabs), and the mixture was reacted at 37°C for 30 minutes.

### 1-2. Transcription reaction using T7 polymerase

As a conventionally known method, the ligated product in Example 1-1 was added to 10x T7 polymerase reaction buffer [40 mM Tris-HCl (pH 7.9), 6 mM MgCl₂, 1 mM DTT, 10 mM NaCl, 2 mM Spermidine] with 25 mM NTPs, 10 mM DTT, and RNase Inhibitor (Takara Bio Inc), and the mixture was reacted at 37°C for 16 hours.

### 1-3. Fluorescence reaction through linkage of downstream aptamer sequence with malachite green

The RNA (with an aptamer sequence) generated in Example 1-2 was added to be 1 µM, and then was mixed to malachite green aptamer reaction buffer [50 mM Tris-HCl (pH 7.5), 1 mM ATP, 10 mM NaCl, 140 mM KCl]. Then, refolding was performed by denaturing at 95°C for 10 minutes in a conventionally known method. Then, the RNA solution was cooled to room temperature for 20 minutes. MgCh was added to the RNA solution to a final concentration of 10 mM, and the solution was stabilized at room temperature for 15 minutes. After that, 5 µL of an aqueous solution of malachite green (320 µM) was added to the target material. The malachite green aqueous solution was used by diluting malachite green oxalic acid salt (Sigma-Aldrich., St. Louis, USA) in RNase-free water. Thereafter, fluorescence was measured in a Hidex Sense Microplate Reader at a volume of 100 µL in 96 well black polystyrene microplate clear flat bottom (Corning Inc., New York, USA) (excitation: 616 nm, emission: 665 nm). A series of reactions in Example 1 described above is shown in FIG. 1C.

### 1-4. Result

This Example used an RNA aptamer that binds to malachite green and expresses fluorescence to detect a target gene, thereby detecting a transcription product generated from the ligated probe.

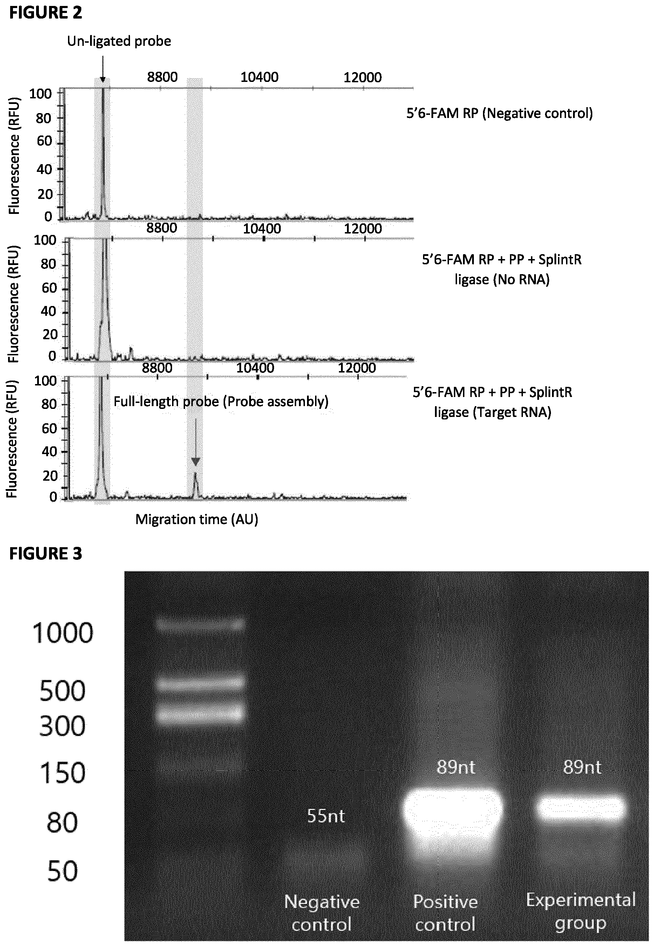

In order to verify whether the designed probe performed a ligation reaction through SplintR ligase, the reaction product was confirmed by an electrophoresis method. At this time, capillary electrophoresis [ABI 3130XL Genetic Analyzer (16-Capillary Array, 50 cm; Applied Biosystems Inc., Foster City, USA)] was used and analyzed for sensitive detection of the ligated probe assembly (full-length probe). First, an oligonucleotide with 6-FAM (6-Carboxyfluorescein) attached to 5' of RP was prepared. The 5'6-FAM RP showed a peak at a specific position regardless of whether or not a ligation reaction occurs. A peak appeared at a different position in the ligated sample. Because SplintR ligase connects two probes only when the target RNA is present, the ligation reaction occurs only in the sample including the target RNA, so that the peak of a ligated probe assembly in which two probes were connected was generated at different positions from the peak of 5'6-FAM RP (FIG. 2).

Thereafter, the position of the band was confirmed by agarose gel electrophoresis in order to determine whether the probe assembly was properly transcribed. The gel used was a 2.5% agarose gel denatured gel, and a sample without target RNA was used as a negative control, and a synthesized probe assembly [Integrated DNA Technologies, INC. (IDT)., Coralville, USA] was set as a positive control. At this time, it was confirmed that since the ligation reaction did not occur in the negative control group, 55 nt transcription product in length from the T7 promoter sequence to PP was made, and that since the ligation reaction proceeded in the positive control group and the experimental group, 89 nt transcription product including both PP and RP was produced from the T7 promoter sequence (FIG. 3).

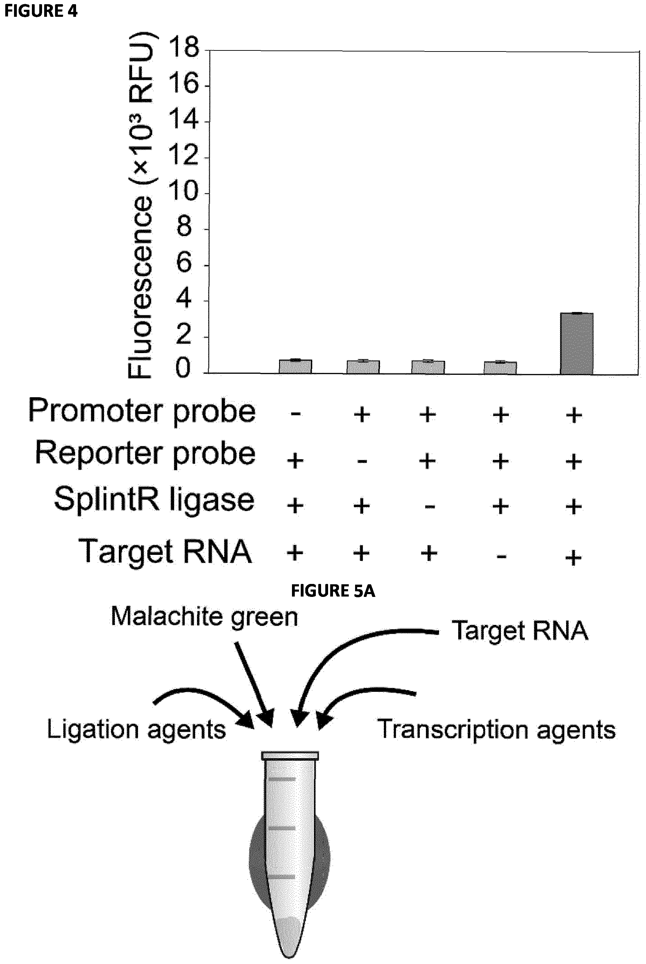

Further, it was confirmed by using the Hidex Sense Microplate Reader (Hidex., Lemminkäisenkatu, Finland) whether the transcription product bound to malachite green to emit fluorescence (FIG. 4).

### Example 2. Construction of one-pot reaction molecular diagnostic platform using ligation reaction

In the process of verifying the molecular diagnostic platform based on the ligation reaction in Example 1, the reactions of Examples 1-1, 1-2, and 1-3 were performed in different tubes under different reaction buffer conditions. Further, the temperature was optimized for each step reaction so that the temperature was not kept constant.

The above processes are quite cumbersome and time-consuming.

Therefore, in order to simply and quickly perform a diagnosis through detection of a target sequence, the present inventors have developed a unified "isothermal one-pot reaction molecular diagnostic method" to proceed with all reactions under a constant temperature condition in one tube using a reaction buffer of a single composition, as shown in FIG. 5A.

Here, the fewer components used, the easier it is to optimize temperature and buffer composition.

Accordingly, the present inventors intentionally attempted to reduce the types of components when designing the detection platform.

In Example 2, the probe set designed in Example 1 (probe set consisting of a sequence that hybridizes to *mecA,* which is a target gene of MRSA as a target RNA; a T7 promoter sequence as a promoter sequence; and a malachite green RNA aptamer sequence region), SplintR ligase, T7 RNA polymerase, and malachite green were used.

### 2-1. Unification of reaction buffer

Each reaction constituting a molecular diagnostic platform using a ligation reaction has an optimal reaction buffer. The component of each reaction buffer known in the art and the component required for the reaction are shown in Table 2 below along with their concentrations.

**[Table 2]**

| Component | SplintR ligase reaction buffer | T7 RNA polymerase reaction buffer | Malachite green reaction buffer |
|---|---|---|---|
| Tris-HCl (mM) | 50 | 40 | 50 |
| MgCl₂ (mM) | 10 | 6 | 10 |
| NTPs (mM) | 0 | 1 | 0 |
| ATP (mM) | 1 | 0 | 1 |
| DTT (mM) | 10 | 1 | 0 |
| NaCl (mM) | 15 | 10 | 5 |
| Spermidine (mM) | 0 | 2 | 0 |

As described above, in order to unify the reaction buffer, 20 candidates of a unified reaction buffer were prepared as a basic component including Tris-HCl, MgClz, NTPs, NaCl, DTT, except ATP, Spermidine, etc., among the components listed in the respective reaction protocols of SplintR ligase and T7 RNA polymerase.

Candidates for the prepared reaction buffers are shown in Table 3 below.

**[Table 3]**

| Candidate | Component |
|---|---|
| 1 | 50 mM Tris-HCl, 10 mM MgCl₂, 1 mM NTPs, 10.5 mM NaCl |
| 2 | 50 mM Tris-HCl, 10 mM MgCl₂, 1 mM NTPs, 13 mM NaCl |
| 3 | 50 mM Tris-HCl, 10 mM MgCl₂, 1 mMNTPs, 15.5 mM NaCl |
| 4 | 50 mM Tris-HCl, 10 mM MgCl₂, 1 mM NTPs, 18 mM NaCl |
| 5 | 50 mM Tris-HCl, 10 mM MgCl₂, 1 mM NTPs, 10.5 mM NaCl, 1.25 mM DTT |
| 6 | 50 mM Tris-HCl, 10 mM MgCl₂, 1 mM NTPs, 13 mM NaCl, 1.25 mM DTT |
| 7 | 50 mM Tris-HCl, 10 mM MgCl₂, 1 mMNTPs, 15.5 mMNaCl, 1.25 mM DTT |
| 8 | 50 mM Tris-HCl, 10 mM MgCl₂, 1 mM NTPs, 18 mM NaCl, 1.25 mM DTT |
| 9 | 50 mM Tris-HCl, 10 mM MgCl₂, 1 mM NTPs, 10.5 mM NaCl, 2.5 mM DTT |
| 10 | 50 mM Tris-HCl, 10 mM MgCl₂, 1 mM NTPs, 13 mM NaCl, 2.5 mM DTT |
| 11 | 50 mM Tris-HCl, 10 mM MgCl₂, 1 mMNTPs, 15.5 mMNaCl, 2.5 mM DTT |
| 12 | 50 mM Tris-HCl, 10 mM MgCl₂, 1 mM NTPs, 18 mM NaCl, 2.5 mM DTT |
| 13 | 50 mM Tris-HCl, 10 mM MgCl₂, 1 mM NTPs, 10.5 mM NaCl, 6.25 mM DTT |
| 14 | 50 mM Tris-HCl, 10 mM MgCl₂, 1 mM NTPs, 13 mM NaCl, 6.25 mM DTT |
| 15 | 50 mM Tris-HCl, 10 mM MgCl₂, 1 mMNTPs, 15.5 mMNaCl, 6.25 mM DTT |
| 16 | 50 mM Tris-HCl, 10mM MgCl₂, 1mM NTPs, 18mM NaCl, 6.25mM DTT |
| 17 | 50 mM Tris-HCl, 10 mM MgCl₂, 1 mM NTPs, 10.5 mM NaCl, 12.5 mM DTT |
| 18 | 50 mM Tris-HCl, 10 mM MgCl₂, 1 mM NTPs, 13 mM NaCl, 12.5 mM DTT |
| 19 | 50 mM Tris-HCl, 10 mM MgCl₂, 1 mM NTPs, 15.5 mM NaCl, 12.5 mM DTT |
| 20 | 50 mM Tris-HCl, 10 mM MgCl₂, 1 mM NTPs, 18 mM NaCl, 12.5 mM DTT |

Ligation, transcription, and fluorescence reactions were sequentially performed using the above 20 reaction buffer candidates, and then the intensity of fluorescence was measured. It was checked whether each reaction buffer was suitable for all three reactions and which reaction buffer candidate had the highest reaction efficiency.

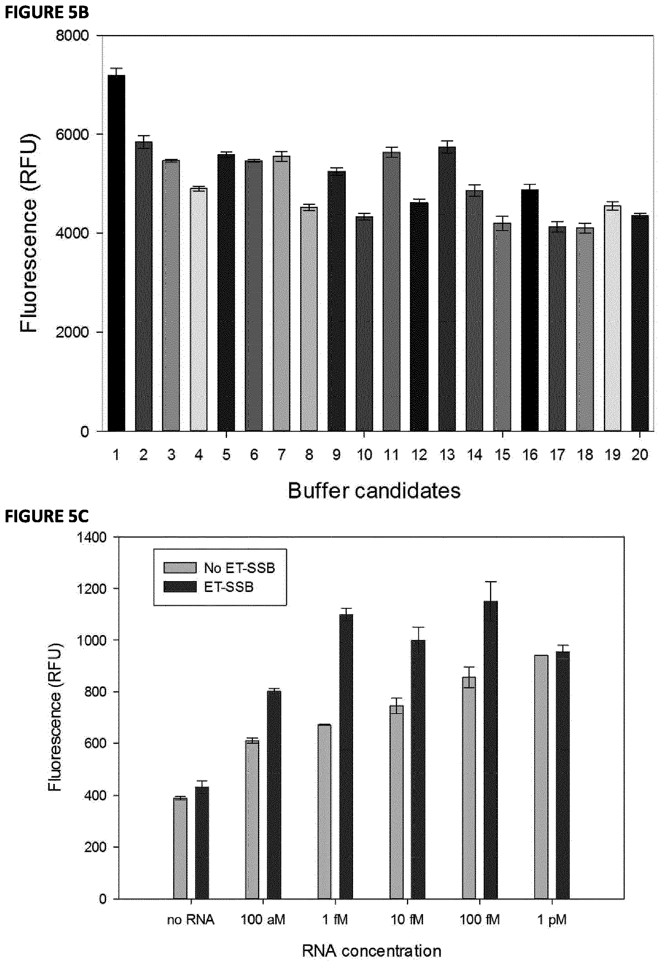

As a result, the measured fluorescence intensity was strongest when reacted with candidate No. 1 consisting of 50 mM Tris-HCl, 10 mM MgCl₂, 1 mM NTPs, and 10.5 mM NaCl from which DTT was removed (FIG. 5B).

Further, as described above, the ligation reaction of PP and RP must occur depending on the presence or absence of splint RNA so that subsequent processes proceed. Thus, the ligation reaction may be said to be the step that determines the precision and accuracy in terms of diagnosis. Further, the efficiency of the ligation reaction determines the efficiency and sensitivity of the diagnosis.

Accordingly, in order to increase the efficiency of the ligation reaction, the present inventors additionally added ET-SSB (extreme thermostable single-stranded DNA binding protein, New England Biolabs) to the reaction buffer No. 1. ET-SSB is known as a protein that stabilizes single-stranded DNA and increases ligation efficiency by reducing off-target effects during ligation reactions. All these proteins are active in the reaction buffer in which the polymerase acts. In view of this, the present inventors measured fluorescence by adding 0.8 µL of ET-SSB (400 ng of ET-SSB to 50 µL reaction volume) based on a reaction volume of 100 µL.

As a result, as shown in FIG. 5C, it was confirmed that the fluorescence of the sample to which ET-SSB was added was higher than that of the sample to which ET-SSB was not added.

As such, the amounts of enzyme, chemical, protein, etc. added for the ligation reaction were optimized to maximize the efficiency of the fluorescence reaction by specifically binding the downstream aptamer sequence and malachite green when the target RNA was present in the "reaction buffer No. 1" of Table 3 prepared above.

Further, it was confirmed that fluorescence was detected even when ligation, transcription, and fluorescence reactions were simultaneously performed in one tube with candidate reaction buffer No. 1 to which ET-SSB was added (FIG. 5D).

As a result, it was confirmed that even if the three-step reaction from ligation, transcription, and fluorescence, which had been progressed in stages, was simultaneously performed in one tube containing the reaction buffer No. 1 to which the ET-SSB of the present invention was added, a series of reactions were performed without any problem.

Hereinafter, in the following Examples, a solution capable of optimally achieving a series of reactions of the present invention, which was prepared by adding ET-SSB to reaction buffer No. 1 (50 mM Tris-HCl, 10 mM MgCl₂, 1 mM NTPs, and 10.5 mM NaCl) was named and used as a "single reaction buffer."

### 2-2. Optimization of the number of molecules required for reaction

Protein including enzymes such as ligase and RNA polymerase and chemicals such as malachite green play a key role in a molecular diagnostic platform using ligation reaction. Accordingly, the present inventors optimized the addition amount of the above-described molecules in order to maximize the intensity of fluorescence in a single reaction buffer.

In this case, SplintR ligase was used as the ligase, and T7 RNA polymerase was used as the RNA polymerase.

First, the present inventors experimented with various combinations in order to optimize the addition amount of SplintR ligase and T7 RNA polymerase.

As a result, through several optimization experiments, the largest fluorescence value was measured when SplintR ligase was 250 units and T7 RNA polymerase was 250 units based on a volume of 100 µL (FIG. 6A).

Further, the present inventors optimized the concentration of malachite green, a chemical molecule, that specifically binds to a downstream aptamer sequence directly related to the fluorescence signal.

As a result of varying the amount of malachite green added, the largest fluorescence value was shown when 16 µM of malachite green was added (FIG. 6B).

### 2-3. Unification of reaction temperature (isothermal reaction)

Since the present invention relates to a single-step reaction using a single reaction buffer, all reactions must occur in one place. For a single-step reaction to occur, the reaction temperature must also be unified. The temperatures at which each reaction is performed are all different from each other in Example 1. The annealing process, the ligation process, and the malachite green reaction, respectively, require a high temperature of 95°C. However, at this temperature, SplintR ligase and T7 RNA polymerase are denatured. Further, most of the reactions constituting molecular diagnosis are all conducted at 37°C.

Accordingly, the present inventors performed all reactions (ligation, transcription, and fluorescence reactions) constituting molecular diagnosis using the single reaction buffer identified above in a single tube at 37°C in order to unify the reaction temperature.

As a result, it was confirmed that a distinguishable level of fluorescence was expressed depending on the presence or absence of the target RNA (FIG. 6C).

The above results demonstrated that a single-step reaction could be performed using a single reaction buffer under isothermal conditions at 37°C.

Hereinafter, in the present invention, the reaction was named "isothermal one-pot reaction," and the composition and concentration of the isothermal one-pot reaction buffer used in this Example are summarized in Table 4 below.

**[Table 4]**

| Component | Concentration |
|---|---|
| PP | 20 nM |
| RP | 22 nM |
| Target RNA | Variable |
| One-pot reaction buffer | 50 mM Tris-HCl, 10 mM MgCl₂, 1 mM NTPs, 10.5 mM NaCl |
| Malachite green | 16 µM |
| ET-SSB | 400 ng |
| RNase Inhibitor | 20 unit |
| Splint R ligase | 250 unit |
| T7 RNA polymerase | 250 unit |

### Example 3. Detection by probe set of present invention in case of using DNA as splint

In Examples 1-1 to 1-3, splint, that is, RNA as a target sequence was used. However, the present inventors additionally used DNA as a target sequence. A ligation reaction between probes using SplintR ligase and a transcription reaction using T7 polymerase were performed under the same conditions and methods as described in Examples 1-1 and 1-2. Then, it was confirmed by a bioanalyzer.

Agilent 2100 was used as the bioanalyzer, and the kit used at this time was Agilent RNA 6000 nano kit. It may sensitively analyze the fragment and size of RNA with the characteristics of electrophoretic separation and microfluidic technology.

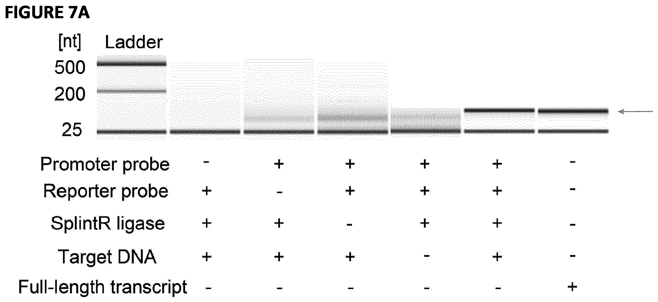

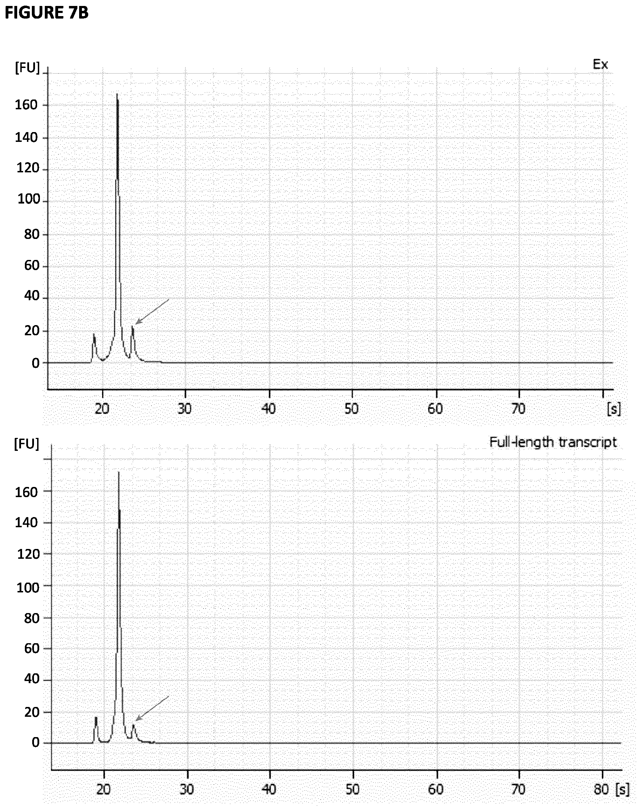

As shown in FIGS. 7A (gel image) and 7B (electropherogram), the results confirmed that target DNA was detected even when DNA was used as a splint.

That is, through the above results, the ligation reaction occurs only when the promoter probe (PP), reporter probe (RP), target DNA, and SplintR ligase are all present, resulting in a probe assembly, and correct transcription of the probe assembly was confirmed by using the synthesized probe assembly as a positive control.

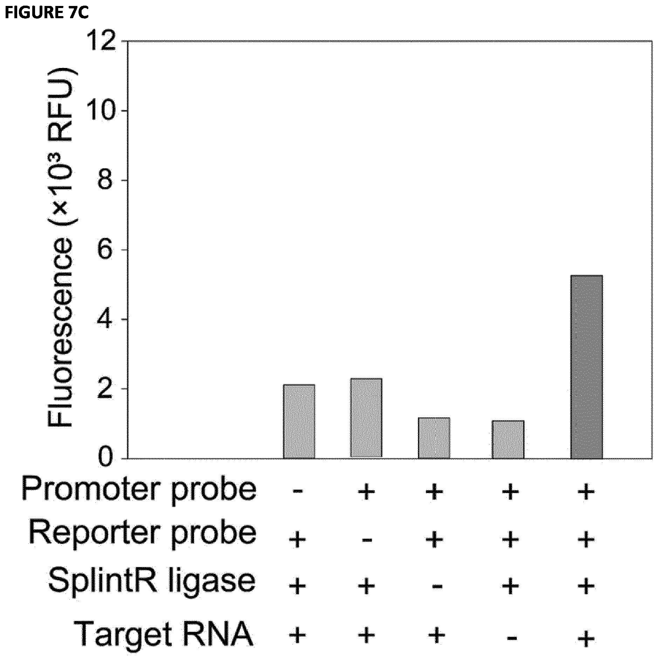

Further, as shown in FIG. 7C, a Hidex Sense Microplate Reader (Hidex., Lemminkäisenkatu, Finland) was used to confirm whether the transcript product bound to malachite green to produce fluorescence.

That is, the above results confirmed that the ligation reaction, the *in vitro* transcription reaction, and the fluorescence expression reaction using the RNA aptamer were all well performed even in the DNA, not the RNA as the splint.

Further, It was confirmed whether the three reactions (ligation reaction, *in vitro transcription* reaction, and fluorescence expression reaction using RNA aptamer) occurred under the "isothermal one-pot reaction" condition identified in Example 2, that is, isothermal at 37°C even when carried out in one vessel with a single reaction buffer containing 50 mM Tris-HCl, 10 mM MgCl₂, 1 mM NTPs, 10.5 mM NaCl and 400 ng of ET-SSB.

DNA of *RdRp* (RNA dependent RNA polymerase), a target gene of SARS-CoV-2 (novel coronavirus), was used as splint DNA, and broccoli aptamer (DFHBI-1T/BRApt) binding to DFHBI-1T ((SZ)-5-[(3,5-Difluoro-4-hydroxyphenyl)methylene]-3,5-dihydro-2-methyl-3-(2,2,2-trifluoroethyl)-4H-imidazol-4-one) was used to detect the target object through a fluorescence reaction between them.

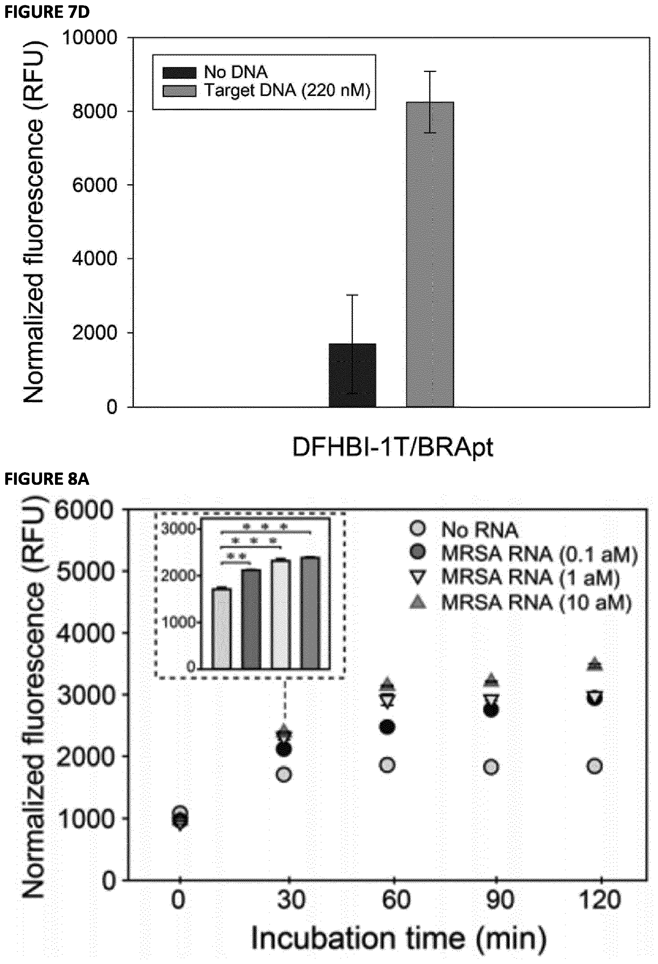

As a result, as shown in FIG. 7D, it can be confirmed that the intensity of fluorescence was higher in the sample including the target DNA than in the state in which the target DNA was not included.

Therefore, it was demonstrated that the molecular diagnostic platform using the ligation reaction of the present invention might be applied to target detection without difficulty even using DNA as a splint.

### Example 4. Confirmation of rapidity, sensitivity and specificity of diagnosis using isothermal one-pot reaction platform

### 4-1. Confirmation of rapidity using isothermal one-pot reaction platform

The present inventors measured the time required for the isothermal one-pot reaction, which was performed in one vessel containing the probe set, the target RNA (*mecA,* the target gene of MRSA as splint RNA), malachite green, ligase and polymerase of the present invention under an isothermal condition of 37°C and single reaction buffer which is the optimum condition of isothermal one-pot reaction for the present invention confirmed in Table 4 of Example 2 above.

At this time, 96-well black polystyrene microplate clear flat bottom (Corning Inc) was used, and the reaction volume was 100 µL.

Different numbers of molecules of target RNA were added. After 0 minutes, 30 minutes, 60 minutes, 90 minutes, and 120 minutes, fluorescence was measured using Hidex Sense Microplate Reader.

As a result, as shown in FIG. 8A, the final result could be quickly obtained in just 2 hours, indicating that the detection time was significantly shortened compared to the PCR method using the conventional amplification reaction.

In particular, it was confirmed that the difference in fluorescence values started to show after 30 minutes, and after a reaction time of 60 minutes, the difference in fluorescence values could be clearly distinguished depending on the presence or absence of the target RNA.

### 4-2. Confirmation of diagnostic sensitivity using isothermal one-pot reaction platform

After revealing that an isothermal one-pot reaction was successfully performed, the limit of detection through this reaction was measured. First, a sample was prepared to have a concentration range of 220 nM to 0.1 aM by stepwise dilution of the target RNA. In terms of the number of molecules of the target RNA to be detected, 220 nM of *mecA* RNA was 253 nt in length, and the RNA contained in 100 µL reaction was 2.2 pmoles, which corresponded to 1.32 × 10¹² RNA molecules.

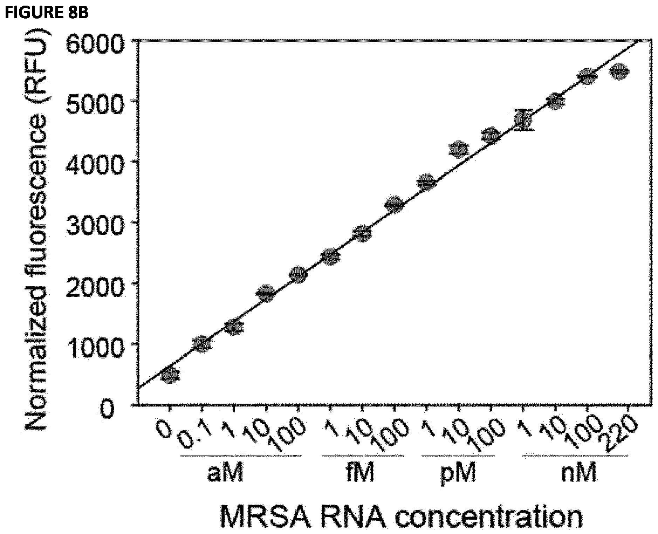

In this Example, target RNAs ranging from 1.32 × 10¹² RNAs (220 nM) to 6 RNAs (0.1 aM) were added, and fluorescence was measured after an isothermal one-pot reaction. Thus, the presence or absence of the target RNA was determined through the difference in fluorescence from the negative control group without target RNA. The present inventors confirmed that up to 6 RNAs could be detected by the isothermal one-pot reaction developed in the present invention (FIG. 8B).

### Example 5. Confirmation of detection of RNA of various pathogens through isothermal one-pot reaction platform

Next, the present inventors reconstructed the isothermal one-pot reaction platform for the detection of RNA markers from various pathogens, and thus demonstrated that the probe for the isothermal one-pot reaction of the present invention was used to quickly, accurately, easily and effectively detect the target of interest with high sensitivity.

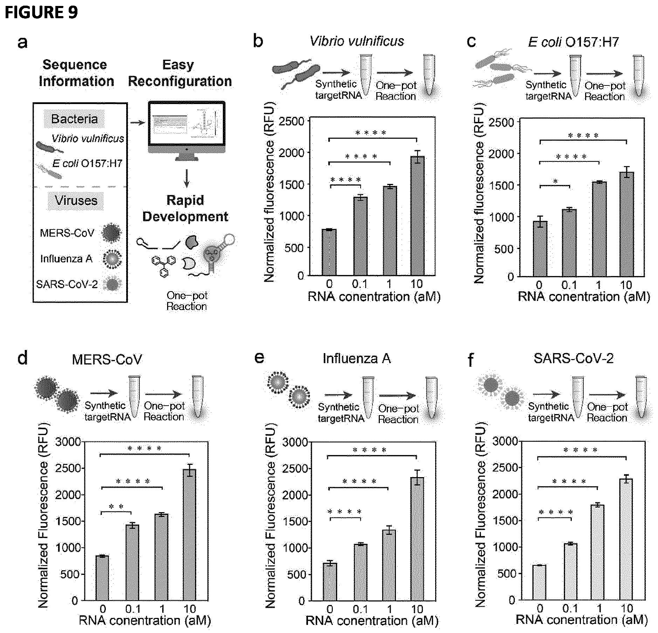

It is only necessary to change the two regions (UHS and DHS) of the probe that hybridizes with the target gene according to the target gene, so that the probe design process may be performed quickly and simply without many calculation steps. Since this isothermal one-pot reaction requires only a nucleotide sequence, it is possible to easily design and construct probes for various infectious diseases (FIG. 9A). If only the sequence of the target gene is secured, the present inventors may easily construct a probe set by adding the desired sequence upstream or downstream. The probe sequences thus prepared are listed in Table 5.

**[Table 5]**

| Pathogen | Type | Sequence (**5'-3'**) | Notes |
|---|---|---|---|
| *Vibrio vulnificus* | MG-PP | TTCTTGTGCGCCAACCTGTAccctatagtgagtc gtattaatttcgcgacaacacgcgaaattaatacgactcactatag gg (SEQ ID No.: 3) | 5'-Ph |
| | MG-RP | ggatccattcgttacctggctctcgccagtcgggatccCTTC TCAACAATCGGCACATA (SEQ ID No.: 4) | |
| *E. coli* O157:H1 | MG-PP | TCAACTCCCCAACGCCTTTTccctatagtgagtc gtattaatttcgcgacaacacgcgaaattaatacgactcactatag gg (SEQ ID No.: 5) | 5'-Ph |
| | MG-RP | ggatccattcgttacctggctctcgccagtcgggatccCGCA CCGCTATTTGACTCCC (SEQ ID No.: 6) | |
| MERS-CoV | MG-PP | AAGAGGAACTGAATCGCGCGccctatagtgag tcgtattaatttcgcgacaacacgcgaaattaatacgactcactata ggg (SEQ ID No.: 7) | 5'-Ph |
| | MG-RP | ggatccattcgttacctggctctcgccagtcgggatccGAGC TCGGGGCGATTATGTG(SEQ ID No.: 8) | |
| Influenza A | MG-PP | TCCCCTGCTCATTGCTATGGccctatagtgagtc gtattaatttcgcgacaacacgcgaaattaatacgactcactatag gg (SEQ ID No.: 9) | 5'-Ph |
| | MG-RP | ggatccattcgttacctggctctcgccagtcgggatccTTTG TCTGCAGCGTATCCAC (SEQ ID No.: 10) | |
| Influenza A | BR-PP | TTCCACAACATACACCCCCTCccctatagtgag tcgtattaatttcgcgacaacacgcgaaattaatacgactcactata ggg (SEQ ID No.: 11) | 5'-Ph |
| | BR-RP | gtatgtgggagacggtcgggtccagatattcgtatctgtcgagt agagtgtgggctcccacatacGGGCGATAAACTCT AGTATGCCA (SEQ ID No.: 12) | |
| SARS-CoV-2(SARS-CoV-MG1) | MG-PP1 | GTTCCACCTGGTTTAACATATAGTccctata gtgagtcgtattaatttcgcgacaacacgcgaaattaatacgactc actatagggg (SEQ ID No.: 13) | 5'-Ph |
| | MG-RP1 | ggatccattcgttacctggctctcgccagtcgggatccGTGG CATCTCCTGATGAG (SEQ ID No.: 14) | |
| SARS-CoV-2(SARS-CoV-MG2) | MG-PP2 | ACACTATTAGCATAAGCAGTTGTGGcccta tagtgagtcgtattaatttcgcgacaacacgcgaaattaatacgac tcactatagggg (SEQ ID No.: 15) | 5'-Ph |
| | MG-RP2 | ggatccattcgttacctggctctcgccagtcgggatccTGAC AGCTTGACAAATGTTAAAA (SEQ ID No.: 16) | |
| SARS-CoV-2(SARS-CoV-BR1) | BR-PP1 | AACACTATTAGCATAAGCAGTTGTGGccc tatagtgagtcgtattaatttcgcgacaacacgcgaaattaatacg actcactatagggg (SEQ ID No.: 17) | 5'-Ph |
| | BR-RP1 | gtatgtgggagacggtcgggtccagatattcgtatctgtcgagt agagtgtgggctcccacatacGTGACAGCTTGACA AATGTTAAA (SEQ ID No.: 18) | |
| SARS-CoV-2(SARS-CoV-BR2) | BR-PP2 | TTTCACTCAATACTTGAGCACACTCATTc cctatagtgagtcgtattaatttcgcgacaacacgcgaaattaatac gactcactatagggg (SEQ ID No.: 19) | 5'-Ph |
| | BR-RP2 | gtatgtgggagacggtcgggtccagatattcgtatctgtcgagt agagtgtgggctcccacatacTAACCGCCACACAT GACCA (SEQ ID No.: 20) | |

| | | | |
|---|---|---|---|
| (MG-RP refers to a reporter probe sequence including a malachite green aptamer sequence. BR-RP refers to a reporter probe sequence including a broccoli aptamer sequence. Nucleotides indicated in uppercase letters refer to the hybridization sequences which are complementary to the target nucleic acid sequence. The nucleotides indicated in lowercase letters refer to the T7 promoter complementary sequence + the loop sequence + the T7 promoter sequence constituting the stem-loop structure, and the underlined nucleotides refer to nucleotides forming the stem in the stem-loop structure. Nucleotides in lowercase italics refer to aptamer sequences (malachite green aptamer; MG, broccoli aptamer sequence; BR). 5'-Ph refers to phosphorylated at the 5'-end.) | | | |

Therefore, in order to demonstrate the detection of various pathogens for isothermal one-pot reaction, two pathogenic microorganisms, *V. vulnificus* (*Vibrio vulnificus*) and *E. coli* O157:H7 (*Escherichia coli* O157:H7) were targeted. *V*. *vulnificus* is known to cause gastroenteritis, wound infection and sepsis in humans. In order to detect this, the present inventors targeted the *vvhA* gene. *vvhA* is a gene having an extracellular cytotoxic effect and hemolytic activity.

As a result, *V. vulnificus* was effectively detected through the isothermal one-pot reaction of the present invention. Further, the sensitivity of the isothermal one-pot reaction using a probe pair for detecting *vvhA* produced through the *in vitro* transcription reaction was 0.1 aM (10-18 mol/L). The linear correlation R2 = 0.9566 between the target gene concentration and the fluorescence intensity was observed (FIG. 9B).

Further, a probe pair was designed to detect *E. coli* O157:H7 causing food poisoning, and *E. coli* O157:H7 was detected using the *tir* gene as a target gene with the probe pair.

As a result, *E. coli* O157:H7 was effectively detected through the isothermal one-pot reaction of the present invention. Similarly, a low RNA concentration of 0.1 aM were detected through the isothermal one-pot reaction. A high linear correlation R2 = 0.9684 between RNA concentration and fluorescence intensity was observed (FIG. 9C).

Further, the target was extended to human infectious RNA viruses that cause lethal disease.

First, the middle east respiratory syndrome coronavirus (MERS-CoV) was targeted. The mortality rate of MERS-CoV has been reported to be 35% and can be transmitted from person to person, necessitating rapid and sensitive point-of-care testing.

As a result, MERS-CoV was effectively detected through the isothermal one-pot reaction of the present invention. Further, the probe pair for the MERS-CoV target gene *upE* exhibited similar sensitivity and linearity to the bacterial case (FIG. 9D).

Further, the present inventors designed a probe pair for the influenza A virus target gene, *HA* (hemagglutinin) gene.

As a result, the influenza A virus was effectively detected through the isothermal one-pot reaction of the present invention. Further, similarly, high sensitivity and high linearity were exhibited (FIG. 9E).

Further, a probe pair was designed for a recently emerged virus, severe acute respiratory syndrome coronavirus 2 (SARS-CoV-2) (novel coronavirus). Target gene sequences were selected based on standard real-time polymerase chain reaction (real time-PCR) for SARS-CoV-2 targeting the RNA-dependent RNA polymerase (*RdRp*) gene.

As a result, SARS-CoV-2 was effectively detected through the isothermal one-pot reaction of the present invention. Further, the isothermal one-pot reaction successfully detected a target RNA as low as 0.1 aM (FIG. 9F), demonstrating its high versatility in detecting various target RNAs.

### Example 6. Confirmation of real pathogen detection through isothermal one-pot reaction platform

Next, the present inventors used an isothermal one-pot reaction to detect target RNA from living cells of the pathogen. MRSA, which has already been detected as an isothermal one-pot reaction, was selected as the target pathogen. In order to increase the accuracy of the experiment, methicillin-sensitive *Staphylococcus aureus* (MSSA) without target RNA was used as a negative control. MRSA and MSSA cells were lysed by heating to 95°C to release RNAs. After dilution, it was added to the isothermal one-pot reaction to investigate specificity and sensitivity.

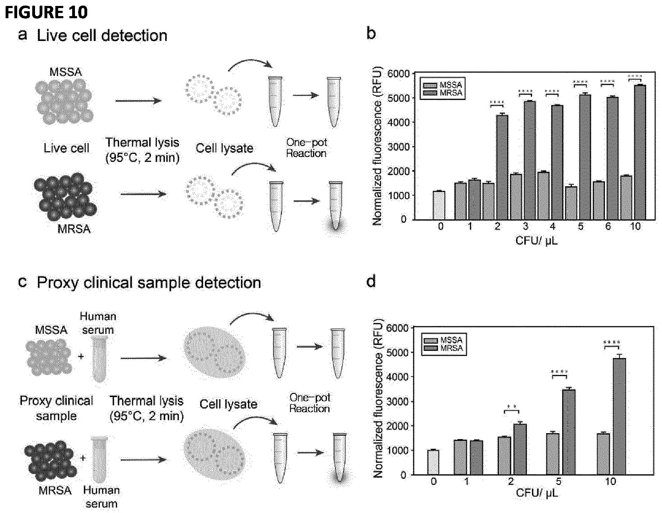

As a result, a significant difference was observed in the fluorescence intensity of the samples containing MRSA and MSSA. The difference in fluorescence between samples containing MRSA and MSSA was clearly shown from 2 CFU (cell forming unit) per 100 µL reaction, indicating high sensitivity and specificity even in the isothermal one-pot reaction using live pathogen samples (FIGS. 10A and 10B).

Finally, diluted MRSA and MSSA pathogen samples were injected into the human serum to further verify the performance of the isothermal one-pot reaction for the detection of target RNA in a state similar to that of a clinical sample (FIG. 10C).

As shown in FIG. 10D, the results confirmed that it was detectable from 2 CFU/µL.

The results confirmed that the isothermal one-pot reaction of the present invention could be applied with high sensitivity and specificity even in actual application fields (similar clinical samples).

### Example 7. Dual target detection using pair of two orthogonal probes of isothermal one-pot reaction

### 7-1. Simultaneous detection of different types of pathogens using isothermal one-pot reaction

The present inventors utilized a simple probe design to extend the function to detect two target RNAs simultaneously in a single isothermal reaction.

It is important to detect multiple biomarkers to make a decision for more accurate target detection by reducing false-positive and false-negative results. Based on the high specificity of isothermal one-pot reaction probes and the availability of RNA aptamers with unique spectral properties, the present inventors have designed two sets of isothermal one-pot reaction probes with orthogonality in a one-pot reaction to detect each target RNA. First, an orthogonal reporter probe for the influenza A virus was developed. Because MRSA infection causes flu-like symptoms, it is necessary to distinguish this pathogen from the common influenza A virus. In addition, influenza A-infected patients are more susceptible to MRSA infection. In summary, the simultaneous detection and identification of both pathogens may aid in diagnosis and follow-up. The orthogonal reporter probe for influenza A virus was designed by replacing its aptamer sequence with the broccoli aptamer sequence binding to DFHBI-1T ((5Z)-5-[(3,5-Difluoro-4-hydroxyphenyl)methylene]-3,5-dihydro-2-methyl-3-(2,2,2-trifluoroethyl)-4H-imidazol-4-one)

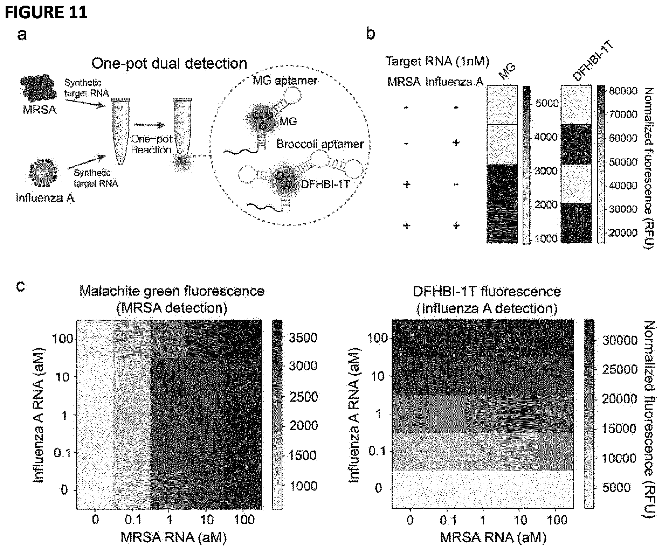

The broccoli aptamer exhibits completely different spectral properties from the malachite green aptamer. The spectral range of malachite green has emission: 616 nm and excitation: 665 nm, whereas that of broccoli aptamer has emission: 460 nm and excitation: 520 nm. NUPACK was used to simulate the secondary structure of the novel reporter probe and the corresponding full-length RNA transcript, which met the probe design criteria by the present inventors without further optimization. Dual detection of MRSA and influenza A virus was carried out as an isothermal one-pot reaction in the addition of two pairs of probes, a fluorescent dye to which they bind, and various concentrations of target RNA (FIG. 11A). When the probe pairs are hybridized to their respective target RNAs followed by a successful transcription reaction, the RNA aptamers bind to the fluorescent dye to which they bind to emit distinguishable fluorescence. The presence of respective target RNAs may be determined by the fluorescence pattern from the isothermal one-pot reaction: malachite green aptamer fluorescence for MRSA and broccoli aptamer fluorescence for influenza A virus. In other words, the presence of target RNA (1 nM) was readily detected by different fluorescence patterns (FIG. 11B). Over various concentrations of respective target RNAs, the isothermal one-pot reaction probes confirmed that fluorescence responding only to the respective target was specifically generated (FIG. 11C). The present inventors verified that the isothermal one-pot reaction was used to allow the dual detection for the two pathogens.

### 7-2. Simultaneous detection of multiple target sites of specific pathogens using isothermal one-pot reaction

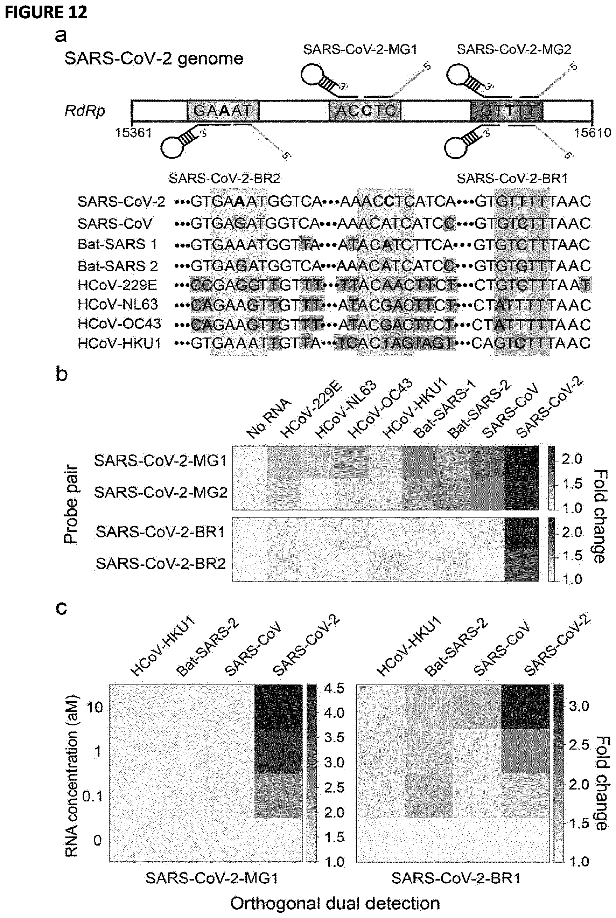

The present inventor applied orthogonal dual detection to SARS-CoV-2. Simultaneous detection of multiple target sites according to the genome may distinguish this pathogen from many related viruses with high sequence homology. In addition to the previously demonstrated (see FIG. 9F) probe pairs, three additional probe pairs were designed for different regions of the *RdRp* gene using malachite green aptamer (MG) or broccoli aptamer (BR). Each probe pair contains mismatched bases at the 5'-end of the promoter probe (PP) or the 3'-end of the reporter probe (RP). This is only present in SARS-CoV-2 compared to other similar viruses (FIG. 12A). Since hybridization does not occur between the probe and non-target RNAs, subsequent reactions including ligation, transcription, and fluorescence do not occur, enabling specific detection of SARS-CoV-2. In fact, these four probe pairs may detect 1 aM of SARS-CoV-2 target RNA and exhibit higher fluorescence intensity compared to the related viral RNA sequence (FIG. 12B). Then, the SARS-CoV-2-MG1 and SARS-CoV-2-BR2 probe pairs were used to test whether orthogonal dual detection of the two target regions was successfully performed.

As a result, it was confirmed that each of the different regions of the target RNA was effectively detected by dual detection of the isothermal one-pot reaction (FIG. 12C).

Therefore, dual detection using an isothermal one-pot reaction may be used for more accurate diagnosis by providing two complementary detection results.

Hereinbefore, specific parts of the present invention have been described in detail. These specific descriptions are only preferred embodiments for those of ordinary skill in the art. It is clear that the scope of the present invention is not limited thereby. Accordingly, it is intended that the substantial scope of the present invention be defined by the appended claims and their equivalents.

## Claims

1. A probe set of an isothermal one-pot reaction for detecting a target nucleic acid sequence, the probe set comprising a first probe and a second probe,
wherein the first probe is a promoter probe (PP) having a structure represented by the following general formula I;
3'-X-Y-5' (I)
wherein, X represents a stem-loop structure portion having a promoter sequence that may be recognized by RNA polymerase; Y represents an upstream hybridization sequence (UHS) portion having a hybridization sequence complementary to the target nucleic acid sequence; the target nucleic acid sequence is DNA or RNA; and X and Y are deoxyribonucleotides;
wherein the second probe is a reporter probe (RP) having a structure of represented by the following general formula II;
3'-Y'-Z-5' (II)
wherein Y' represents a downstream hybridization sequence (DHS) portion having a hybridization sequence complementary to the target nucleic acid sequence; Z represents an aptamer sequence portion having a label or an interactive label system containing a plurality of labels to generate a detectable signal; the target nucleic acid sequence is DNA or RNA; and Y' and Z are deoxyribonucleotides;
wherein the first probe and the second probe are positioned at immediately adjacent locations to each other when hybridized with the target nucleic acid sequence;
wherein the first probe and the second probe are hybridized with the target nucleic acid sequence to allow ligation of the first probe and the second probe; transcription of the ligation product is initiated by RNA polymerase to generate a signal; and
wherein the ligation is performed by SplintR ligase.

2. The probe set of claim 1, wherein the RNA polymerase is selected from the group consisting of bacteriophage T7 RNA polymerase, bacteriophage T3 polymerase, bacteriophage RNA polymerase, bacteriophage θII polymerase, *Salmonella* bacteriophage SP6 polymerase, *Pseudomonas* bacteriophage gh-1 polymerase, *E. coli* RNA polymerase holoenzyme, *E. coli* RNA polymerase core enzyme, human RNA polymerase I, human RNA polymerase II, human RNA polymerase III, human mitochondrial RNA polymerase and variants thereof.

3. The probe set of claim 1, wherein the label is selected from the group consisting of a chemical label, an enzymatic label, a radioactive label, a fluorescent label, a luminescent label, a chemiluminescent label, and a metal label.

4. The probe set of claim 1, wherein the isothermal one-pot reaction is simultaneously performed in one vessel at any one of a unified temperatures in the range of 15°C to 50°C without a separate amplification reaction; and wherein the isothermal one-pot reaction is simultaneously performed with a unified one-pot reaction buffer containing Tris-HCl, MgCl₂, NTPs, NaCl and ET-SSB (extreme thermostable single-stranded DNA binding protein).

5. A composition for detecting a target nucleic acid sequence, the composition comprising: the probe set of an isothermal one-pot reaction comprising the first probe and the second probe of claim 1; a ligation agent, an RNA polymerase and an isothermal one-pot reaction buffer.

6. The composition of claim 5, wherein the composition comprises two or more probe sets of an isothermal one-pot reaction for detecting target nucleic acid sequences.

7. The composition of claim 6, wherein each of the two or more probe sets of an isothermal one-pot reaction for detecting target nucleic acid sequences comprises different interactive label system; each of the two or more probe sets binds to different target nucleic acid sequence; whereby the probe sets allow multiple detecting the different target nucleic acid sequences.

8. The composition of claim 5, wherein the isothermal one-pot reaction buffer comprises Tris-HCl, MgCl₂, NTPs, NaCl and ET-SSB (extreme thermostable single-stranded DNA binding protein); and the isothermal one-pot reaction is simultaneously performed with a unified single reaction buffer.

9. A kit for detecting a target nucleic acid sequence, the kit comprising the composition for detecting the target nucleic acid sequence according to any one of claims 6 to 8.

10. A method for detecting a target nucleic acid sequence under an isothermal one-pot reaction conditions without a separate amplification reaction, the method comprising following steps of:
(a) treating a sample with the probe set of an isothermal one-pot reaction for detecting a target nucleic acid sequence comprising the first probe and the second probe of claim 1 to hybridize with the target nucleic acid sequence;
(b) treating the hybridization product of step (a) with a ligation agent to ligate the first probe and the second probe of the probe set, and treating the ligation product with a polymerase to initiate transcription; and
(c) treating the transcription product of step (b) with an aptamer-reactive substance to detect aptamer signal generation in the transcription product, wherein the signal generation indicates presence of the target nucleic acid sequence in the sample.

11. The method of claim 10, wherein the isothermal one-pot reaction is simultaneously performed in one vessel at any one of a unified temperatures in the range of 15°C to 50°C without the separate amplification reaction; and wherein the isothermal one-pot reaction is simultaneously performed with a unified one-pot reaction buffer containing Tris-HCl, MgCh, NTPs, NaCl and ET-SSB (extreme thermostable single-stranded DNA binding protein).

12. A molecular diagnostic method for testing at on-site under an isothermal one-pot reaction condition, without a separate amplification reaction, the method comprising following steps of:
(a) treating a sample with the probe set of an isothermal one-pot reaction for detecting a target nucleic acid sequence comprising the first probe and the second probe of claim 1 to hybridize with the target nucleic acid sequence;
(b) treating the hybridization product of step (a) with a ligation agent to ligate the first probe and the second probe of the probe set, and treating the ligation product with a polymerase to initiate transcription; and
(c) treating the transcription product of step (b) with an aptamer-reactive substance to detect aptamer signal generation in the transcription product, wherein the signal generation indicates presence of the target nucleic acid sequence in the sample.

13. The method of claim 12, wherein the isothermal one-pot reaction is simultaneously performed in one vessel at any one of a unified temperatures in the range of 15°C to 50°C without the separate amplification reaction; and wherein the isothermal one-pot reaction is simultaneously performed with a unified one-pot reaction buffer containing Tris-HCl, MgCh, NTPs, NaCl and ET-SSB (extreme thermostable single-stranded DNA binding protein).

14. The method of claim 12, wherein the target is a pathogenic microorganism; and the pathogenic microorganism is at least one selected from the group consisting of *Staphylococcus Aureus, Vibrio vulnificus, E. coli,* Middle East Respiratory Syndrome Coronavirus, Influenza A virus, Severe Acute Respiratory Syndrome Coronavirus, respiratory syncytial virus (RSV), human immunodeficiency virus (HIV), herpes simplex virus (HSV), human papillomavirus (HPV), human parasite influenza virus (HPIV), dengue virus, hepatitis B virus (HBV), yellow fever virus, rabies virus, *Plasmodium,* cytomegalovirus (CMV), *Mycobacterium tuberculosis, Chlamydia trachomatis,* Rotavirus, human metapneumovirus (hMPV), Crimean-Congo hemorrhagic fever virus, Ebola virus, Zika virus, Henipavirus, Norovirus, Lassavirus, Rhinovirus, Flavivirus, Rift valley fever virus, hand-foot-and-mouth disease virus, *Salmonella* sp., *Shigella* sp., *Enterobacteriaceae* sp., *Pseudomonas* sp., *Moraxella* sp., *Helicobacter* sp. and *Stenotrophomonas* sp.

## Patentansprüche

1. Sondensatz einer isothermen Eintopfreaktion zum Nachweis einer Ziel-Nukleinsäuresequenz, wobei der Sondensatz eine erste Sonde und eine zweite Sonde umfasst,
wobei die erste Sonde eine Promotorsonde (PP) mit einer Struktur ist, die durch die folgende allgemeine Formel I dargestellt wird;
3'-X-Y-5' (I)
wobei X einen Stamm-Schleifen-(stem loop-)Strukturabschnitt mit einer Promotorsequenz darstellt, die durch RNA-Polymerase erkannt werden kann; Y einen Upstream-Hybridisierungssequenz-(UHS-)Abschnitt darstellt, der eine zur Ziel-Nukleinsäuresequenz komplementäre Hybridisierungssequenz aufweist; die Ziel-Nukleinsäuresequenz DNA oder RNA ist; und X und Y Desoxyribonukleotide sind;
wobei die zweite Sonde eine Reportersonde (RP) mit einer Struktur ist, die durch die folgende allgemeine Formel II dargestellt wird;
3'-Y'-Z-5' (II)
wobei Y' einen Downstream-Hybridisierungssequenz-(DHS-)Abschnitt darstellt, der eine zur Ziel-Nukleinsäuresequenz komplementären Hybridisierungssequenz aufweist; Z einen Aptamer-Sequenzabschnitt mit einer Markierung oder einem interaktiven Markierungssystem darstellt, das mehrere Markierungen enthält, um ein nachweisbares Signal zu erzeugen; die Ziel-Nukleinsäuresequenz DNA oder RNA ist; und Y' und Z Desoxyribonukleotide sind;
wobei die erste Sonde und die zweite Sonde bei der Hybridisierung mit der Ziel-Nukleinsäuresequenz an unmittelbar benachbarten Stellen zueinander positioniert sind;
wobei die erste Sonde und die zweite Sonde mit der Ziel-Nukleinsäuresequenz hybridisiert werden, um Ligation der ersten Sonde und der zweiten Sonde zu ermöglichen; die Transkription des Ligationsprodukts durch RNA-Polymerase initiiert wird, um ein Signal zu erzeugen; und
wobei die Ligation durch SplintR-Ligase durchgeführt wird.

2. Sondensatz nach Anspruch 1, wobei die RNA-Polymerase ausgewählt ist aus der Gruppe bestehend aus Bakteriophagen-T7-RNA-Polymerase, Bakteriophagen-T3-Polymerase, Bakteriophagen-RNA-Polymerase, Bakteriophagen-811-Polymerase, *Salmonella*-Bakteriophagen-SP6-Polymerase, *Pseudomonas-Bakteriophagen-gh-1-*Polymerase, *E. coli*-RNA-Polymerase-Holoenzym, *E. coli*-RNA-Polymerase-Kemenzym, menschlicher RNA-Polymerase I, menschlicher RNA-Polymerase II, menschlicher RNA-Polymerase III, menschlicher mitochondrialer RNA-Polymerase und Varianten davon.

3. Sondensatz nach Anspruch 1, wobei die Markierung aus der Gruppe bestehend aus einer chemischen Markierung, einer enzymatischen Markierung, einer radioaktiven Markierung, einer fluoreszierenden Markierung, einer lumineszierenden Markierung, einer chemolumineszierenden Markierung und einer Metallmarkierung ausgewählt ist.

4. Sondensatz nach Anspruch 1, wobei die isotherme Eintopfreaktion gleichzeitig in einem Gefäß bei einer einheitlichen Temperatur im Bereich von 15 °C bis 50 °C ohne separate Amplifikationsreaktion durchgeführt wird;
und wobei die isotherme Eintopfreaktion gleichzeitig mit einem einheitlichen Eintopf-Reaktionspuffer durchgeführt wird, der Tris-HCl, MgCh, NTPs, NaCl und ET-SSB (extrem thermostabiles einzelsträngiges DNA-Bindungsprotein; extreme thermostable single-stranded DNA binding protein) enthält.

5. Zusammensetzung zum Nachweis einer Ziel-Nukleinsäuresequenz, wobei die Zusammensetzung umfasst: den Sondensatz einer isothermen Eintopfreaktion, umfassend die erste Sonde und die zweite Sonde nach Anspruch 1; ein Ligationsmittel, eine RNA-Polymerase und einen isothermen Eintopf-Reaktionspuffer.

6. Zusammensetzung nach Anspruch 5, wobei die Zusammensetzung zwei oder mehr Sondensätze einer isothermen Eintopfreaktion zum Nachweis von Ziel-Nukleinsäuresequenzen umfasst.

7. Zusammensetzung nach Anspruch 6, wobei jeder der zwei oder mehr Sondensätze einer isothermen Eintopfreaktion zum Nachweis von Ziel-Nukleinsäuresequenzen unterschiedliche interaktive Markierungssysteme umfasst; jeder der zwei oder mehr Sondensätze an unterschiedliche Ziel-Nukleinsäuresequenzen bindet; wobei die Sondensätze den mehrfachen Nachweis der unterschiedlichen Ziel-Nukleinsäuresequenzen ermöglichen.

8. Zusammensetzung nach Anspruch 5, wobei der isotherme Eintopf-Reaktionspuffer Tris-HCl, MgCh, NTPs, NaCl und ET-SSB (extrem thermostabiles einzelsträngiges DNA-Bindungsprotein; extreme thermostable single-stranded DNA binding protein) umfasst; und die isotherme Eintopfreaktion gleichzeitig mit einem einheitlichen Einzelreaktionspuffer durchgeführt wird.

9. Kit zum Nachweis einer Ziel-Nukleinsäuresequenz, wobei das Kit die Zusammensetzung zum Nachweis der Ziel-Nukleinsäuresequenz nach einem der Ansprüche 6 bis 8 umfasst.

10. Verfahren zum Nachweis einer Ziel-Nukleinsäuresequenz unter isothermen Eintopfreaktionsbedingungen ohne separate Amplifikationsreaktion, wobei das Verfahren die folgenden Schritte umfasst:
(a) Behandeln einer Probe mit dem Sondensatz einer isothermen Eintopfreaktion zum Nachweis einer Ziel-Nukleinsäuresequenz umfassend die erste Sonde und die zweite Sonde nach Anspruch 1, um mit der Ziel-Nukleinsäuresequenz zu hybridisieren;
(b) Behandeln des Hybridisierungsprodukts aus Schritt (a) mit einem Ligationsmittel, um die erste Sonde und die zweite Sonde des Sondensatzes zu ligieren, und Behandeln des Ligationsprodukts mit einer Polymerase, um die Transkription zu initiieren; und
(c) Behandeln des Transkriptionsprodukts aus Schritt (b) mit einer Aptamerreaktiven Substanz, um Aptamer-Signalerzeugung im Transkriptionsprodukt nachzuweisen, wobei die Signalerzeugung das Vorhandensein der Ziel-Nukleinsäuresequenz in der Probe anzeigt.

11. Verfahren nach Anspruch 10, wobei die isotherme Eintopfreaktion gleichzeitig in einem Gefäß bei einer einheitlichen Temperatur im Bereich von 15°C bis 50°C ohne die separate Amplifikationsreaktion durchgeführt wird; und wobei die isotherme Eintopfreaktion gleichzeitig mit einem einheitlichen Eintopf-Reaktionspuffer durchgeführt wird, der Tris-HCl, MgCh, NTPs, NaCl und ET-SSB (extrem thermostabiles einzelsträngiges DNA-Bindungsprotein; extreme thermostable single-stranded DNA binding protein) enthält.

12. Molekulardiagnostisches Verfahren zum Testen vor Ort unter einer isothermen Eintopfreaktion ohne separate Amplifikationsreaktion, wobei das Verfahren die folgenden Schritte umfasst:
(a) Behandeln einer Probe mit dem Sondensatz einer isothermen Eintopfreaktion zum Nachweis einer Ziel-Nukleinsäuresequenz umfassend die erste Sonde und zweite Sonde nach Anspruch 1, um mit der Ziel-Nukleinsäuresequenz zu hybridisieren;
(b) Behandeln des Hybridisierungsprodukts aus Schritt (a) mit einem Ligierungsmittel, um die erste Sonde und die zweite Sonde des Sondensatzes zu ligieren, und Behandeln des Ligationsprodukts mit einer Polymerase, um die Transkription zu initiieren; und
(c) Behandeln des Transkriptionsprodukts von Schritt (b) mit einer Aptamerreaktiven Substanz, um Aptamer-Signalerzeugung im Transkriptionsprodukt nachzuweisen, wobei die Signalerzeugung das Vorhandensein der Ziel-Nukleinsäuresequenz in der Probe anzeigt.

13. Verfahren nach Anspruch 12, wobei die isotherme Eintopfreaktion gleichzeitig in einem Gefäß bei einer einheitlichen Temperatur im Bereich von 15°C bis 50°C ohne die separate Amplifikationsreaktion durchgeführt wird; und wobei die isotherme Eintopfreaktion gleichzeitig mit einem einheitlichen Eintopf-Reaktionspuffer durchgeführt wird, der Tris-HCl, MgCh, NTPs, NaCl und ET-SSB (extrem thermostabiles einzelsträngiges DNA-Bindungsprotein; extreme thermostable single-stranded DNA binding protein) enthält.

14. Verfahren nach Anspruch 12, wobei das Ziel ein pathogener Mikroorganismus ist; und der pathogene Mikroorganismus mindestens einer ist ausgewählt aus der Gruppe bestehend aus *Staphylococcus Aureus, Vibrio vulnificus, E. coli,* Middle East Respiratory Syndrome Coronavirus, Influenza A-Virus, Severe Acute Respiratory Syndrome Coronavirus, Respiratory Syncytial Virus (RSV), Human Immunodeficiency Virus (HIV), Herpes-simplex-Virus (HSV), humanem Papillomavirus (HPV), humanem Parasiten-Influenzavirus (HPIV), Dengue-Virus, Hepatitis-B-Virus (HBV), Gelbfiebervirus, Tollwutvirus, *Plasmodium,* Cytomegalovirus (CMV), *Mycobacterium tuberculosis, Chlamydia trachomatis,* Rotavirus, humanem Metapneumovirus (hMPV), hämorrhagischem Krim-Kongo-Fieber-Virus, Ebola-Virus, Zika-Virus, Henipavirus, Norovirus, Lassavirus, Rhinovirus, Flavivirus, Rift-Valley-Fieber-Virus, Hand-Fuß-Mund-Krankheits-Virus, *Salmonella* sp., *Shigella* sp., *Enterobacteriaceae* sp., *Pseudomonas* sp., *Moraxella* sp., *Helicobacter* sp. und *Stenotrophomonas* sp.

## Revendications

1. Jeu de sondes d'une réaction monotope isotherme pour détecter une séquence d'acide nucléique cible, le jeu de sondes comprenant une première sonde et une deuxième sonde,
dans lequel la première sonde est une sonde de promoteur (PP) ayant une structure représentée par la formule générale I suivante ;
3'-X-Y-5' (I)
dans laquelle X représente une portion de structure tige-boucle ayant une séquence de promoteur qui peut être reconnue par une ARN polymérase ; Y représente une portion de séquence d'hybridation en amont (UHS) ayant une séquence d'hybridation complémentaire de la séquence d'acide nucléique cible ; la séquence d'acide nucléique cible est un ADN ou un ARN ; et X et Y sont des désoxyribonucléotides ;
dans lequel la deuxième sonde est une sonde rapporteur (RP) ayant une structure représentée par la formule générale II suivante ;
3'-Y'-Z-5' (II)
dans laquelle Y' représente une portion de séquence d'hybridation en aval (DHS) ayant une séquence d'hybridation complémentaire de la séquence d'acide nucléique cible ; Z représente une portion de séquence d'aptamère ayant un marqueur ou un système marqueur interactif contenant une pluralité de marqueurs pour générer un signal détectable ; la séquence d'acide nucléique cible est un ADN ou un ARN ; et Y' et Z sont des désoxyribonucléotides ;
dans lequel la première sonde et la deuxième sonde sont positionnées à des positions immédiatement adjacentes l'une de l'autre quand elles sont hybridées avec la séquence d'acide nucléique cible ;
dans lequel la première sonde et la deuxième sonde sont hybridées avec la séquence d'acide nucléique cible pour permettre une ligature de la première sonde et de la deuxième sonde ; la transcription du produit de ligature est initiée par une ARN polymérase pour générer un signal ; et
dans lequel la ligature est effectuée par la ligase SplintR.

2. Jeu de sondes selon la revendication 1, dans lequel l'ARN polymérase est choisie dans le groupe constitué par une ARN polymérase de bactériophage T7, une polymérase de bactériophage T3, une ARN polymérase de bactériophage, une polymérase de bactériophage θII, une polymérase de bactériophage de *Salmonella* SP6, une polymérase de bactériophage de *Pseudomonas* gh-1, une holoenzyme d'une ARN polymérase d'*E*. *coli,* une enzyme de coeur d'une ARN polymérase d'*E*. *coli,* une ARN polymérase I humaine, une ARN polymérase II humaine, une ARN polymérase III humaine, une ARN polymérase mitochondriale humaine, et leurs variants.

3. Jeu de sondes selon la revendication 1, dans lequel le marqueur est choisi dans le groupe constitué par un marqueur chimique, un marqueur enzymatique, un marqueur radioactif, un marqueur fluorescent, un marqueur luminescent, un marqueur chimioluminescent, et un marqueur métallique.

4. Jeu de sondes selon la revendication 1, dans lequel la réaction monotope isotherme est simultanément effectuée dans un seul récipient à l'une quelconque de températures unifiées situées dans la plage allant de 15 °C à 50 °C sans réaction d'amplification séparée ; et dans lequel la réaction monotope isotherme est simultanément effectuée avec un tampon réactionnel monotope unifié contenant Tris-HCl, MgCl₂, des NTPs, NaCl et ET-SSB (protéine de liaison à un ADN simple brin thermostable extrême).

5. Composition pour détecter une séquence d'acide nucléique cible, la composition comprenant : le jeu de sondes d'une réaction monotope isotherme comprenant la première sonde et la deuxième sonde de la revendication 1 ; un agent de ligature, une ARN polymérase et un tampon réactionnel monotope isotherme.

6. Composition selon la revendication 5, laquelle composition comprend deux ou plus de deux jeux de sondes d'une réaction monotope pour détecter des séquences d'acides nucléiques cibles.

7. Composition selon la revendication 6, dans laquelle chacun des deux ou plus de deux jeux de sondes d'une réaction monotope isotherme pour détecter des séquences d'acides nucléiques cibles comprend un système de marqueur interactif différent ; chacun des deux ou plus de deux jeux de sondes se lie à une séquence d'acide nucléique cible différente ; moyennant quoi les jeux de sondes permettent une détection multiple des différentes séquences d'acides nucléiques cibles.

8. Composition selon la revendication 5, dans laquelle le tampon réactionnel monotope isotherme comprend Tris-HCl, MgCl₂, des NTPs, NaCl et ET-SSB (protéine de liaison à un ADN simple brin thermostable extrême) ; et la réaction monotope isotherme est simultanément effectuée avec un tampon réactionnel unique unifié.

9. Trousse pour détecter une séquence d'acide nucléique cible, la trousse comprenant la composition pour détecter une séquence d'acide nucléique cible selon l'une quelconque des revendications 6 à 8.

10. Méthode pour détecter une séquence d'acide nucléique cible dans des conditions réactionnelles monotopes isothermes sans réaction d'amplification séparées, la méthode comprenant les étapes suivantes :
(a) traitement d'un échantillon avec le jeu de sondes d'une réaction monotope isotherme pour détecter une séquence d'acide nucléique cible comprenant la première sonde et la deuxième sonde de la revendication 1 pour s'hybrider avec la séquence d'acide nucléique cible ;
(b) traitement du produit d'hybridation de l'étape (a) avec un agent de ligature pour ligaturer la première sonde et la deuxième sonde du jeu de sondes, et traitement du produit de ligature avec une polymérase pour initier une transcription ; et
(c) traitement du produit de transcription de l'étape (b) avec une substance réactive aux aptamères pour détecter la génération d'un signal d'aptamère dans le produit de transcription, dans lequel la génération de signal indique la présence de la séquence d'acide nucléique cible dans l'échantillon.

11. Méthode selon la revendication 10, dans laquelle la réaction monotope isotherme est simultanément effectuée dans un seul récipient à l'une quelconque de températures unifiées situées dans la plage allant de 15 °C à 50 °C sans réaction d'amplification séparée ; et dans laquelle la réaction monotope isotherme est simultanément effectuée avec un tampon réactionnel monotope unifié contenant Tris-HCl, MgCl₂, des NTPs, NaCl et ET-SSB (protéine de liaison à un ADN simple brin thermostable extrême).

12. Méthode de diagnostic moléculaire pour un test sur site dans des conditions réactionnelles monotopes isothermes, sans réaction d'amplification séparée, la méthode comprenant les étapes suivantes :
(a) traitement d'un échantillon avec le jeu de sondes d'une réaction monotope isotherme pour détecter une séquence d'acide nucléique cible comprenant la première sonde et la deuxième sonde de la revendication 1 pour s'hybrider avec la séquence d'acide nucléique cible ;
(b) traitement du produit d'hybridation de l'étape (a) avec un agent de ligature pour ligaturer la première sonde et la deuxième sonde du jeu de sondes, et traitement du produit de ligature avec une polymérase pour initier une transcription ; et
(c) traitement du produit de transcription de l'étape (b) avec une substance réactive aux aptamères pour détecter la génération d'un signal d'aptamère dans le produit de transcription, dans lequel la génération de signal indique la présence de la séquence d'acide nucléique cible dans l'échantillon.

13. Méthode selon la revendication 12, dans laquelle la réaction monotope isotherme est simultanément effectuée dans un seul récipient à l'une quelconque de températures unifiées situées dans la plage allant de 15 °C à 50 °C sans réaction d'amplification séparée ; et dans laquelle la réaction monotope isotherme est simultanément effectuée avec un tampon réactionnel monotope unifié contenant Tris-HCl, MgCl₂, des NTPs, NaCl et ET-SSB (protéine de liaison à un ADN simple brin thermostable extrême).

14. Méthode selon la revendication 12, dans laquelle la cible est un microorganisme pathogène ; et le microorganisme pathogène est au moins l'un choisi dans le groupe constitué par *Staphylococcus aureus, Vibrio vulnificus, E. coli,* le coronavirus du syndrome respiratoire du Moyen-Orient, le virus grippal A, le coronavirus du syndrome respiratoire aigu sévère, un virus respiratoire syncytial (VRS), un virus de l'immunodéficience humaine (VIH), un virus herpès simplex (VHS), un papillomavirus humain (PVH), un virus para-influenza humain (HPIV), un virus de la dengue, un virus d'hépatite B (VHB), le virus de la fièvre jaune, le virus de la rage, *Plasmodium,* le cytomégalovirus (CMV), *Mycobacterium tuberculosis, Chlamydia trachomatis,* un rotavirus, un métapneumovirus humain (MPVh), le virus de la fièvre hémorragique de Crimée-Congo, le virus Ebola, le virus Zika, un hénipavirus, un norovirus, un lassavirus, un rhinovirus, un flavivirus, le virus de la fièvre de la vallée du Rift, le virus de la maladie mains-pieds-bouche, *Salmonella* sp., *Shigella* sp., *Enterobacteriaceae* sp., *Pseudomonas* sp., *Moraxella* sp., *Helicobacter* sp. et *Stenotrophomonas* sp.
